(19) Europäisches Patentamt
European Patent Office
Office européen des brevets

(11) EP 4 209 506 A1

(12) **EUROPEAN PATENT APPLICATION**
published in accordance with Art. 153(4) EPC

(43) Date of publication:
**12.07.2023 Bulletin 2023/28**

(21) Application number: **21864357.5**

(22) Date of filing: **01.09.2021**

(51) International Patent Classification (IPC):
***C07K 16/00*** (2006.01) ***C12N 5/10*** (2006.01)
***C12N 1/15*** (2006.01) ***C12N 1/19*** (2006.01)
***C12N 1/21*** (2006.01) ***C12N 15/54*** (2006.01)
***C12N 15/56*** (2006.01) ***C12N 15/63*** (2006.01)
***C12N 9/10*** (2006.01) ***C12N 9/24*** (2006.01)
***C12P 21/02*** (2006.01) ***C12P 21/08*** (2006.01)

(52) Cooperative Patent Classification (CPC):
**C07K 16/00; C12N 5/10; C12N 9/10; C12N 9/24; C12N 15/63; C12N 15/74; C12N 15/80; C12N 15/81; C12P 21/02**

(86) International application number:
**PCT/JP2021/032083**

(87) International publication number:
**WO 2022/050300 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.09.2020 JP 2020147745**

(71) Applicant: **Daiichi Sankyo Company, Limited**
**Tokyo 103-8426 (JP)**

(72) Inventors:
- **NISHIZAWA Hanako**
  **Tokyo 134-8630 (JP)**
- **ONO Yasunori**
  **Tokyo 134-8630 (JP)**
- **IWAMOTO Mitsuhiro**
  **Tokyo 103-8426 (JP)**

(74) Representative: **Marks & Clerk LLP**
**15 Fetter Lane**
**London EC4A 1BW (GB)**

Remarks:
The complete document including Reference Tables and the Sequence Listing can be downloaded from the EPO website

(54) **NOVEL ENDO-?-N-ACETYLGLUCOSAMINIDASE**

(57) The present invention provides endo-β-N-acetylglucosaminidase (Endo-Si) cloning from a strain belonging to *Streptococcus iniae and* a mutant enzyme thereof, a gene encoding the enzyme, a recombinant plasmid, a transformant obtained by transformation of a cell by the plasmid and use thereof, and a method for producing e.g., a sugar chain remodeled antibody using the enzyme. A polypeptide having an amino acid sequence at amino acid positions 34 to 928 in SEQ ID NO: 2 or an amino acid sequence having the same amino acid sequence except containing one or mutations at more amino acid positions selected from the group consisting of amino acids at position 241 (D241), 190 (T190), 311 (Q311) and 360 (E360), said polypeptide exhibiting a sugar chain hydrolysis activity and/or transglycosylation activity.

EP 4 209 506 A1

## Description

Technical Field

The present invention relates to endo-β-N-acetylglucosaminidase (Endo-Si), a gene encoding the enzyme, a recombinant plasmid, a transformant obtained by transformation of a cell by the plasmid and use thereof, a method for producing an antibody such as a sugar chain-remodeled antibody by using the enzyme, etc.

Background Art

Antibodies are glycoprotein molecules having an N-linked sugar chain (N297-linked sugar chain) linked to the side chain of Asn at position 297 located in the Fc region of a heavy chain molecule thereof. The antibody is an important molecule in basic research and the field of medicine. In particular, research/development of antibodies as antibody drugs has been intensively carried out and various effects of a sugar chain have been elucidated (Non-Patent Literature 1). Medical-use antibodies mostly employed at present are IgG-class molecules. Such antibodies are usually produced by cultured animal cells represented by CHO cells and NS0 cells. The N297-linked sugar chains of these antibodies produced by animal cells are biantennary complex-type sugar chains but they have heterogeneity in core fucose, terminal sialic group, galactosyl group and bisecting GlcNAc (Non-Patent Literature 2). It has been found that the N297-linked sugar chain of an antibody has a large effect on effector activity including ADCC activity (antibody-dependent cell-mediated cytotoxicity) and CDC activity (complement-dependent cytotoxicity) of the antibody (Non-Patent Literature 3 and Non-Patent Literature 4), and it has been pointed out that the sugar chain may influence the blood half-life of the antibody (Non-Patent Literature 5). It has also been found that an antibody having an N297-linked sugar chain the non-reducing end of which has been 2,6-sialylated serves as a main medicinal ingredient (active ingredient) of IVIG (intravenous immunoglobulin) (Non-Patent Literature 6). In the molecules for medical use containing IgG and an Fc fragment, it is considered that the heterogeneity of the N297-linked sugar chain greatly affects the characteristics and quality of the antibody serving as an active ingredient. Thus, it cannot be denied that contamination with a trace amount of a modified molecule with a sugar chain having heterogeneity may greatly change the characteristics of a final product.

In the circumstances, in producing a medical-use antibody and a glycoprotein molecule containing an antibody Fc region, techniques for obtaining homogeneous sugar chains are being developed. As a method for homogeneously producing a sugar chain to be added to a glycoprotein, a transglycosylation reaction using an enzyme has been known (Non-Patent Literatures 7 to 9). The transglycosylation reaction is a multiple step process carried out *in vitro* and consisting of cleaving a sugar chain (hydrolysis reaction) and adding of another sugar chain by condensation (transglycosylation reaction). In particular, for conversion of an N-linked sugar chain, an enzyme family called endo-β-N-acetylglucosaminidase (ENGase) is used. The required characteristics of endo-β-N-acetylglucosaminidase (ENGase) are: 1) having an ability to hydrolyze a complex-type sugar chain in a substrate specific manner and 2) having an ability to perform a transglycosylation reaction to a predetermined structure. As the transglycosylation reaction, the following methods are known: a method of transferring an oxazolylated sugar chain at reducing end to a GlcNAc (N-acetylglucosamine) acceptor by use of a single ENGase (Non-Patent Literatures 7 and 8) and a one-pot method of directly transferring a sugar chain to a GlcNAc acceptor by use of two types of ENGases (Non-Patent Literature 9 and Patent Literature 1). ENGases have been isolated from various biological species and a wild type ENGase or a mutant ENGase are selectively used depending on the type of sugar chain prefered as a substrate.

Examples of ENGases commonly known in the technical field include Endo-A (enzyme derived from *Arthrobacter protophormiae* ) (Non-Patent Literature 10), Endo-D (enzyme derived from *Streptococcus pneumoniae-derived*) (Non-Patent Literature 11), Endo-M (enzyme derived from *Mucor hiemalis*) (Non-Patent Literature 12), Endo-H (Non-Patent Literature 13), Endo-F2 (enzyme derived from *Flavobacterium meningosepticum*), Endo-F3 (enzyme derived from *Flavobacterium meningosepticum*) (Non-Patent Literature 14), Endo-E (enzyme derived from *Enterococcus faecalis*) (Non-Patent Literature 15), Endo-S (enzyme derived from *Streptococcus pygenes*) (Non-Patent Literature 16), Endo-Tsp1006 (derived from bacteria of the genus *Tannerella*), Endo-Tsp1263 (derived from bacteria of the genus *Tannerella*), Endo-Bno1263 (derived from bacteria of the genus *Bacteroides*), Endo-Tsp1457 (derived from bacteria of the genus *Tannerella*), Endo-Bac1008 (derived from bacteria of the genus *Muribaculum*), Endo-Tsp1603 (derived from bacteria of the genus *Tannerella*), Endo-Tsp1263 (derived from bacteria of the genus *Tannerella*) (Patent Literature 2), Endo-β-N-acetylglucosaminidase derived from the bacteria of the genus *Sphingobacterium* (ORF1152), Endo-β-N-acetylglucosaminidase derived from the bacteria of the genus *Sphingobacterium* (ORF1188)), Endo-β-N-acetylglucosaminidase derived from the bacteria of the genus *Sphingobacterium* (ORF3046), Endo-β-N-acetylglucosaminidase derived from the bacteria of the genus *Sphingobacterium* (ORF3750), Endo-β-N-acetylglucosaminidase derived from filamentous fungi of the genus *Cordyceps,* Endo-β-N-acetylglucosaminidase from filamentous fungi of the genus *Beauveria* (Non-Patent Literature 17), Endo-CC1 enzyme derived from *Coprinopsis cinerea*), Endo-CC2 (enzyme derived from *Coprinopsis cinerea*) (Non-Patent Literature 18), Endo-Om (enzyme derived from *Ogataea minuta*) (Non-Patent Literature 19), Endo-CE (enzyme

derived from *Caenorhabditis elegans*) (Non-Patent Literature 20), Endo-BH (enzyme derived from *Bacillus halodurans* C-125) (Non-Patent Literature 21), EndoSd (enzyme derived from *Streptococcus dysgalactiae*), EndoS2d (enzyme derived from *Streptococcus dysgalactiae*) (Non-Patent Literature 22), EndoSe (enzyme derived from *Streptococcus equi* subsp. *equi*) (Non-Patent Literature 23), Endo-Rp (enzyme derived from *Rhizomucor pusillus*) (Patent Literature 3), EndoS2 and EndoS49 (Non-Patent Literature 24).

Of these ENGases, EndoS (Non-Patent Literature 25), EndoS2 (Non-Patent Literature 26) and Endo-F3 (Non-Patent Literature 27) are known as the enzymes which prefer, as a substrate, a complex N297-linked sugar chain having the core fucose of an antibody and which have been confirmed to have both hydrolysis activity and transglycosylation activity.

As to EndoS, its mutant enzyme, EndoS D233Q, is known to have limited hydrolysis activity to some extent and selectively mediate a transglycosylation reaction in the condition where an intermediate oxazolylated at the reducing end of sugar chain is present in a large amount in the reaction system (Patent Literature 4 and Non-Patent Literature 8).

It is also known that if another mutation is introduced to EndoS D233Q, the mutant EndoS D233Q acquires higher transglycosylation activity or lower hydrolytic activity than an original EndoS D233Q (Patent Literature 5).

It is known that EndoS2 mutant enzyme (D184Q) exhibits higher transglycosylation activity and lower hydrolysis activity than wild type EndoS2 enzyme (Patent Literatures 6 to 8 and Non-Patent Literature 28).

It is known that Endo-F3 mutant enzyme (D165A or D165Q) exhibits lower hydrolysis activity on a product and higher transglycosylation activity of sugar-oxazoline than wild type Endo-F3 enzyme. Endo-F3 mutant enzyme can use bi-and tri- antennary glycosyl oxazolines as substrates for glycosyltransferase (Non-Patent Literature 27).

As the ENGase used in a one-pot method, in which a sugar chain is directly transferred to a GlcNAc acceptor, a combination use of two enzymes, for example, a combination of EndoS/EndoS mutant enzyme and Endo-M/Endo-M mutant enzyme or Endo-CC/Endo-CC mutant enzyme, is known (Patent Literature 1 and Non-Patent Literature 9).

*Streptococcus iniae* (Non-Patent Literature 29) is known as a fish pathogen giving serious damage to the aquacultures of flounder and red sea bream in Japan (Non-Patent Literature 30). To deal with this, a flounder β-hemolytic streptococcal inactivated vaccine, "M back iniae" (Matsuken Pharmaceutical Industry Co., Ltd.) is sold. Classification of *Streptococcus* bacterial strains, which are pathogenic bacteria seriously affecting humans and agriculture, forestry and fisheries, have been carried out based on genomic analysis (Non-Patent Literature 31). Although ENGase sequence of *S. iniae* has been found, the enzyme activity thereof has not been investigated (Non-Patent Literature 24).

Citation List

Patent Literatures

Patent Literature 1: WO2018/003983
Patent Literature 2: JP2020-022440
Patent Literature 3: WO2018/101451
Patent Literature 4: WO2013/120066
Patent Literature 5: WO2017/010559
Patent Literature 6: WO2017/124084
Patent Literature 7: WO2018/039373
Patent Literature 8: JP2020-500549


Non-Patent Literatures

Non-Patent Literature 1: Arnold JN, et al., Annu Rev Immunol. 2007, 25, 21-50
Non-Patent Literature 2: Jefferis R, Biotechnol Prog. 2005, 21, 11-16
Non-Patent Literature 3: Nimmerjahn F, et al., Nat Rev Immunol. 2008, 8, 34-47
Non-Patent Literature 4: Jefferis R, Nat Rev Drug Discov. 2009, 8, 226-234
Non-Patent Literature 5: Bumbaca D, et al., AAPS J. 2012, 14, 554-558
Non-Patent Literature 6: Anthony RM, et al., Science. 2008, 320, 373-376
Non-Patent Literature 7: Wang LX, Trends Glycosci Glycotechnol. 2011, 23, 33-52
Non-Patent Literature 8: Huang W, et al., J Am Chem Soc. 2012, 134, 12308-12318
Non-Patent Literature 9: Iwamoto M, et al., PLoS ONE 2018, 13, e0193534
Non-Patent Literature 10: Takegawa K, et al., Biochem Int. 1991, 24, 849-855

Non-Patent Literature 11: Fan SQ, et al., J Biol Chem. 2012, 287, 11272-11281
Non-Patent Literature 12: Yamamoto K, et al., Biochem Biophys Res Commun. 1994, 203, 244-252
Non-Patent Literature 13: Robbins PW, et al., J Biol Chem. 1984, 259, 7577-7583
Non-Patent Literature 14: Huang W, et al., Chembiochem. 2011, 12, 932-941
Non-Patent Literature 15: Collin M and Fischetti VA. J Biol Chem. 2004, 279, 22558-22570
Non-Patent Literature 16: Collin M and Olsen A., EMBO J. 2001, 20, 3046-3055
Non-Patent Literature 17: Huang Y, et al., Sci Rep. 2018, 8, 246
Non-Patent Literature 18: Eshima Y, et al., PLoS One. 2015, 10, e0132859
Non-Patent Literature 19: Murakami S, et al., Glycobiology. 2013, 23, 736-744
Non-Patent Literature 20: Kato T, et al., Glycobiology. 2002, 12, 581-587
Non-Patent Literature 21: Fujita K, et al., Biosci Biotechnol Biochem. 2004, 68, 1059-1066
Non-Patent Literature 22: Shadnezhad A, et al., Future Microbiol. 2016, 11, 721-736
Non-Patent Literature 23: Flock M, et al., Infect Immun. 2012, 80, 2914-2919
Non-Patent Literature 24: Sjogren J, et al., Biochem J. 2013, 455, 107-118
Non-Patent Literature 25: Goodfellow JJ, et al., J Am Chem Soc. 2012, 134, 8030-8033
Non-Patent Literature 26: Shivatare SS, et al., Chem Commun (Camb). 2018, 54, 6161-6164
Non-Patent Literature 27: Giddens JP, et al., J Biol Chem. 2016, 291, 9356-9370
Non-Patent Literature 28: Li T, et al., J Biol Chem. 2016, 291, 16508-16518
Non-Patent Literature 29: Pier GB and Madin SH., Int J Syst Bacteriol. 1976 26, 545-553
Non-Patent Literature 30: Yoshida T., Fish Pathology. 2016, 51, 44-48
Non-Patent Literature 31: Vincent P, et al., Genome Biology and Evolution, 2014, 6, 741-753

Summary of Invention

Technical Problem

An object of the present invention is to provide a novel endo-β-N-acetylglucosaminidase having hydrolysis activity and/or transglycosylation activity on N297-linked sugar chain of a glycoprotein.

Solution to Problem

The present inventors conducted intensive studies with a view to solving the above problem. As a result, they have found that endo-β-N-acetylglucosaminidase (Endo-Si) cloned from a strain belonging to *Streptococcus iniae* has hydrolysis activity on an N297-linked sugar chain; that hydrolysis activity is more lowered by introducing a mutation into Endo-Si; and that Endo-Si has transglycosylation activity of a predetermined level or more. Based on the findings, the present invention has been accomplished.

The present invention (application) provides the following inventions.

[1] A polypeptide having an amino acid sequence at amino acid positions 34 to 928 in SEQ ID NO: 2 or an amino acid sequence having the same amino acid sequence except containing one or more mutations at one or more amino acid positions selected from the group consisting of amino acids at position 241 (D241), position 190 (T190), position 311 (Q311) and position 360 (E360), said polypeptide exhibiting a sugar chain hydrolysis activity and/or transglycosylation activity.

[2] The polypeptide according to [1], having one or more mutations at 1 to 3 amino acid positions selected from the group consisting of amino acids of D241, T190, Q311 and E360.

[3] The polypeptide according to [1] or [2], having one or more mutations selected from the group consisting of the following (A) to (D):

(A) a mutation of the amino acid at position 241 (D241) to glutamine (D241Q), methionine (D241M) or alanine (D241A) in the amino acid sequence of SEQ ID NO: 2;
(B) a mutation of the amino acid at position 190 (T190) to glutamine (T190Q) in the amino acid sequence of SEQ ID NO: 2;
(C) a mutation of the amino acid at position 311 (Q311) to leucine (Q311L) in the amino acid sequence of SEQ ID NO: 2; and
(D) a mutation of the amino acid at position 360 (E360) to glutamine (E360Q), alanine (E360A), asparagine (E360N) or aspartic acid (E360D) in the amino acid sequence of SEQ ID NO: 2.

[4] The polypeptide according to any one of [1] to [3], having one or more mutations selected from the group consisting

of the following (A) to (D):

(A) D241Q or D241M;
(B) T190Q;
(C) Q311L; and
(D) E360Q.

[5] The polypeptide according to any one of [1] to [4], comprising the following amino acid sequences (A) to (C):

(A) an amino acid sequence selected from the group consisting of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11;
(B) an amino acid sequence having a homology or identity of at least 90% to each of the amino acid sequences defined in (A) excluding an amino acid at position 241, 190, 311 or 360; or
(C) an amino acid sequence having deletion, substitution and/or addition of one or several amino acids relative to the sequence defined in (A) excluding an amino acid at position 241, 190, 311 or 360.

[6] The polypeptide according to any one of [1] to [5], exhibiting hydrolysis activity and/ transglycosylation activity on an N-linked sugar chain.
[7] The polypeptide according to [6], wherein the N-linked sugar chain is an N-linked sugar chain in a glycoprotein.
[8] The polypeptide according to [6] or [7], wherein the glycoprotein is an antibody or a molecule containing an Fc region of an antibody (an Fc region-containing molecule).
[9] The polypeptide according to any one of [6] to [8], wherein the N-linked sugar chain is an N-linked sugar chain linked to Asn at position 297 of an antibody (an N297-linked sugar chain).
[10] The polypeptide according to [9], wherein the N297-linked sugar chain is a complex-type sugar chain having a non-reducing end optionally modified chemically.
[11] The polypeptide according to [9] or [10], wherein the N297-linked sugar chain is an N297-linked sugar chain optionally having fucose added to core GlcNAc.
[12] A polynucleotide encoding the polypeptide according to any one of [1] to [11].
[13] An expression vector comprising the polynucleotide according to [12].
[14] A host cell transformed with the expression vector according to [13].
[15] A production method for the polypeptide according to any one of [1] to [11], comprising a step of culturing the host cell according to [14] and a step of collecting a polypeptide of interest from a culture obtained in the culturing step.
[16] A polypeptide obtained by the production method according to [15].
[17] A production method for an antibody or its Fc region-containing molecule, comprising reacting an acceptor molecule, which is an antibody or its Fc region-containing molecule having, as an N297-linked sugar chain, core GlcNAc optionally having fucose added therefore, with a sugar-chain donor molecule containing GlcNAc having a reducing end activated, in the presence of the polypeptide according to any one of [1] to [11].
[18] The production method according to [17], wherein the GlcNAc having a reducing end activated is oxazolylated GlcNAc.
[19] The production method according to [17] or [18], wherein the sugar-chain donor molecule is a complex-type sugar chain having a non-reducing end optionally modified chemically.
[20] The production method according to any one of [17] to [19], wherein the sugar-chain donor molecule is SG(10)-Ox, MSG1(9)-Ox, MSG2(9)-Ox or a mixture of MSG1(9)-Ox and MSG2(9)-Ox, having a non-reducing end optionally modified chemically.
[21] The production method according to any one of [17] to [20], wherein the sugar-chain donor molecule is [$N_3$-PEG(3)]$_2$-SG(10)-Ox, [$N_3$-PEG(3)]-MSG1(9)-Ox, [$N_3$-PEG(3)]-MSG2(9)-Ox or a mixture of [$N_3$-PEG(3)]-MSG1(9)-Ox and [$N_3$-PEG(3)]-MSG2(9)-Ox.
[22] The production method according to [21], further comprising a step of reacting the azide group ($N_3$-) with a molecule having an alkyne structure.
[23] The production method according to [22], wherein the molecule having an alkyne structure is selected from a chemotherapeutic agent, a molecular target drug, an immunostimulant, a toxin, an antibacterial agent, an antiviral agent, a diagnostic agent, a protein, a peptide, an amino acid, a nucleic acid, an antigen, a lipid, a liposome, a vitamin and a hormone.
[24] The production method according to [23], wherein the chemotherapeutic agent is selected from camptothecin, pyrrolobenzodiazepine, doxorubicin, auristatin, taxane and a derivative thereof.
[25] The production method according to [23], wherein the immunostimulant is selected from a STING agonist, a TLR agonist, an A2AR antagonist, an IDO inhibitor, an antagonist to any one of CTLA-4, LAG-3 and PD-1 pathways, a checkpoint inhibitor, a vascular endothelial growth factor (VEGF) receptor inhibitor, a smoothen inhibitor, an

alkylating agent, an antimetabolite, retinoid, an anticancer vaccine and an adjuvant.

[26] The production method according to any one of [23] to [25], wherein the molecule having an alkyne structure is selected from the group consisting of (A) to (E):

(A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;

(B) N-[4-(11,12-didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10' (5'H)-carbonyl]oxy}methyl)phenyl]-L-alaninamide;

(C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;

(D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide; and

(E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R, 12aR, 14R, 15R, 15aR, 16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2$\lambda^5$, 10$\lambda^5$-furo[3,2-1][1,3,6,9,11,2,10]pentaoxadiphosphacyclotetradecyn-7-yl]-6-oxo-6,9-dihydro-1H-purin-1-yl}ethoxy)methyl]glycinamide.

[27] The production method according to any one of [17] to [26], wherein the acceptor molecule is an antibody or Fc region-containing molecule having an N297-linked sugar chain consisting of core GlcNAc optionally having fucose added.

[28] A production method for an antibody or an Fc region-containing molecule, comprising reacting an acceptor molecule, which is an antibody or its Fc region-containing molecule having, as an N297-linked sugar chain, core GlcNAc optionally having fucose added, with a sugar-chain donor molecule containing GlcNAc having a reducing end not activated, in the presence of the polypeptide according to any one of [1] to [11] and endo-β-N-acetylglucosaminidase (sometimes referred to as Enzyme A) that prefers a complex-type sugar chain of a sugar-chain donor molecule having a reducing end not activated as a substrate but does not prefer an N297-linked sugar chain as a substrate.

[29] The production method according to [27], comprising reacting the polypeptide according to any one of [1] to [11], Enzyme A, the acceptor molecule and the sugar-chain donor molecule in a single reaction solution.

[30] The production method according to [28] or [29], wherein the sugar-chain donor molecule is a complex-type sugar chain having a non-reducing end optionally modified chemically.

[31] The production method according to any one of [28] to [30], wherein the sugar-chain donor molecule is SGP, (SG-)Asn, (MSG1-)Asn, (MSG2-)Asn or a mixture of (MSG1-)Asn and (MSG2-)Asn, having a non-reducing end optionally modified chemically.

[32] The production method according to any one of [28] to [31], wherein the sugar-chain donor molecule is ([$N_3$-PEG(3)]$_2$-SG-)Asn-PEG(3)-$N_3$, ([$N_3$-PEG(3)]-MSG1-)Asn-PEG(3)-$N_3$, ([$N_3$-PEG(3)]-MSG2-)Asn-PEG(3)-$N_3$ or a mixture of ([$N_3$-PEG(3)]-MSG1-)Asn-PEG(3)-$N_3$ and ([$N_3$-PEG(3)]-MSG2-)Asn-PEG(3)-$N_3$.

[33] The production method according to [32], further comprising a step of reacting the azide group ($N_3$-) with a molecule having an alkyne structure.

[34] The production method according to [33], wherein the molecule having an alkyne structure is selected from a chemotherapeutic agent, a molecular target drug, an immunostimulant, a toxin, an antibacterial agent, an antiviral agent, a diagnostic agent, a protein, a peptide, an amino acid, a nucleic acid, an antigen, a lipid, a liposome, a vitamin and a hormone.

[35] The production method according to [34], wherein the chemotherapeutic agent is selected from camptothecin, pyrrolobenzodiazepine, doxorubicin, auristatin, taxane and a derivative thereof.

[36] The production method according to [34], wherein the immunostimulant is selected from a STING agonist, a TLR agonist, an A2AR antagonist, an IDO inhibitor, an antagonist to any one of CTLA-4, LAG-3 and PD-1 pathways,

a checkpoint inhibitor, a vascular endothelial growth factor (VEGF) receptor inhibitor, a smoothen inhibitor, an alkylating agent, an antimetabolite, retinoid, an anticancer vaccine and an adjuvant.

[37] The production method according to any one [34] to [36], wherein the molecule having an alkyne structure is selected from the group consisting of (A) to (E):

(A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;

(B) N-[4-(11,12-didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10' (5'H)-carbonyl]oxy}methyl) phenyl]-L-alaninamide;

(C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;

(D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide; and

(E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R, 12aR, 14R, 15R, 15aR, 16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2$\lambda^5$,10$\lambda^5$-furo[3,2-1][1,3,6,9,11,2,10]pentaoxadiphosphacyclotetradecyn-7-yl]-6-oxo-6,9-dihydro- 1H-purin-1-yl}ethoxy)methyl]glycinamide.

[38] The production method according to any one of [28] to [37], wherein the acceptor molecule is an antibody or Fc region-containing molecule having an N297-linked sugar chain consisting of core GlcNAc optionally having fucose added.

[39] The production method according to any one of [28] to [38], wherein Enzyme A is an enzyme having transglycosylation activity from SGP to an acceptor having GlcNAc.

[40] The production method according to any one of [28] to [39], wherein Enzyme A is any one of Endo-M, Endo-Rp, Endo-Om, Endo-CC and a mutant enzyme thereof having a reduced hydrolysis activity.

[41] The production method according to [40], wherein the mutant enzyme having a reduced hydrolysis activity is selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-CC N180H and Endo-Om N194Q.

[42] An antibody or Fc region-containing molecule obtained by the production method according to any one of [17] to [41].

[43] A production method for an antibody or Fc region-containing molecule having only core GlcNAc optionally having fucose added, comprising reacting an antibody or Fc region-containing molecule with a polypeptide having an amino acid sequence at amino acid positions 34 to 928 in SEQ ID NO: 2.

[44] An antibody or Fc region-containing molecule having only core GlcNAc obtained by the production method according to [43].

The description incorporates the contents disclosed in Japanese Patent Application No. 2020-147745 based on which the priority of the present application claims.

Advantageous Effects of Invention

The Endo-Si enzyme of the present invention has satisfactory hydrolysis activity and acts on an N-linked sugar chain containing N297 link of a glycoprotein to efficiently cut a β-1,4-glycosidic bond between GlcNAc molecules of a core chitobiose structure present in a sugar chain. The sugar chain separated can be used as a sample for analyzing

the sugar chain structure of a glycoprotein and as a raw material for a sugar chain derivative. If a glycoprotein is used as a substrate, a glycoprotein from which a sugar chain is hydrolyzed can be used as an acceptor molecule for sugar chain remodeling.

Since Endo-Si mutant enzyme prepared by introducing a mutation into Endo-Si has lower hydrolysis activity and higher transglycosylation activity than wild type Endo-Si, an antibody or a sugar chain-containing molecule (including an Fc region-containing molecule) having homogenous sugar chains can be obtained by sugar chain remodeling, efficiently or with high purity. Further, the molecular weight of a sugar chain donor to be used in remodeling a sugar chain can be reduced, so that a production cost for an antibody or sugar chain-containing molecule having a remodeled sugar chain can be reduced.

Brief Description of Drawings

[Figure 1] The figure shows the structural formula of [$N_3$-PEG(3)]-MSG1(9)-Ox.
[Figure 2] The figure shows the structural formula of SGP.
[Figure 3] The figure is a graph showing changes in hydrolysis activity of Endo-Si (o) and Endo-S (×) on trastuzumab (mAb 1) with time. The X-axis represents time after initiation of a reaction and the Y axis represents the sugar chain hydrolysis rate.
[Figure 4] The figure shows a schematic illustration of a hydrolysis reaction to the N297-linked sugar chain of an antibody by Endo-Si or an Endo-Si mutant enzyme.
[Figure 5] The figure shows a schematic illustration of a transglycosylation reaction using Endo-Si or an Endo-Si mutant enzyme when a sugar-chain oxazolylated was used as a donor.
[Figure 6] The figure shows the sequence (Endo-Si nucleotide sequence) of SEQ ID NO: 1.
[Figure 7] The figure shows the sequence (Endo-Si amino acid sequence) of SEQ ID NO: 2.
[Figure 8] The figure shows the sequence (Endo-Si amino acid sequence D241Q) of SEQ ID NO: 3.
[Figure 9] The figure shows the sequence (Endo-Si amino acid sequence D241Q/Q311L) of SEQ ID NO: 4.
[Figure 10] The figure shows the sequence (Endo-Si amino acid sequence D241Q/E360Q) of the SEQ ID NO: 5.
[Figure 11] The figure shows the sequence (Endo-Si amino acid sequence D241M) of SEQ ID NO: 6.
[Figure 12] The figure shows the sequence (Endo-Si amino acid sequence D241M/Q311L) of SEQ ID NO: 7.
[Figure 13] The figure shows the sequence (Endo-Si amino acid sequenceD241M/E360Q) of SEQ ID NO: 8.
[Figure 14] The figure shows the sequence (Endo-Si amino acid sequence T190Q/D241Q) of SEQ ID NO: 9.
[Figure 15] The figure shows the sequence (Endo-Si amino acid sequence T190Q) of SEQ ID NO: 10.
[Figure 16] The figure shows the sequence (Endo-Si amino acid sequence T190Q/D241M) of SEQ ID NO: 11.
[Figure 17] The figure shows a schematic view of a transglycosylation reaction by an Endo-Si mutant enzyme and Enzyme A when SGP was used as a donor.
[Figure 18] The figure is a graph showing a relation between the reaction temperatures of Endo-Si and EndoS and the sugar chain hydrolysis rate.
[Figure 19] The figure is a graph showing a relation between reaction pH of Endo-Si and EndoS and the sugar chain hydrolysis rate.
[Figure 20] The figure shows hydrolysis activities of Endo-Si, EndoS and PNGaseF on various types of antibodies in comparison.
[Figure 21] The figure is a schematic illustration of transglycosylation reactions with an Endo-Si mutant enzyme and Enzyme A when ([$N_3$-PEG(3)]-MSG1-)Asn-PEG(3)-$N_3$ was used as a donor.
[Figure 22] The figure is a schematic illustration of transglycosylation reactions with Endo-Si mutant enzyme and Enzyme A when ([$N_3$-PEG(3)]$_2$-SG-)Asn-PEG(3)-$N_3$ was used as a donor.

Description of Embodiments

Now, the present invention will be described below in detail.

In the description, notation of amino acids contained in a molecule follows the practice in this field. A mutation site is indicated by the single-letter code of a wild-type amino acid (or nucleic acid) in combination with the number of position (for example, Asp at position 241 is represented by "D241"). Note that, in the description, a mutation introduced into an amino acid position refers to substitution, deletion, insertion or addition of an amino acid, preferably, substitution of an amino acid. A mutation is expressed as the single-letter code of a wild-type amino acid (or nucleic acid), the number of position and the single-letter code of an amino acid (or nucleic acid) after mutation (for example, in the case of mutation where Asp at position 241 is substituted with Gln, the mutation is expressed as "D241Q"). A (specific) mutant enzyme having a mutation is expressed by molecule (enzyme) name and the mutation site (for example, in the case of a mutant

enzyme where Asp at position 241 of Endo-Si is substituted with Gln, the mutant enzyme is expressed as "Endo-Si D241Q"). In the case where an enzyme has a plurality of mutations, the individual mutations are expressed by putting "/" between them (for example, if Endo-Si D241Q has another mutation where Gln at position 241 is substituted with Leu, the mutant enzyme is expressed by "Endo-Si D241Q/Q311L").

In the present invention, the "N297-linked sugar chain" refers to an N-linked sugar chain linked to the side chain of Asn at position 297 in an IgG heavy chain. If IgG is fragmented and a peptide fragment thereof contains the Asn, the sugar chain linked to the corresponding Asn is included in the N297-linked sugar chain. Usually, an N297-linked sugar chain of IgG produced in, e.g., an animal, has a basic structure represented by the following formula (I) or (II). The non-reducing end of the sugar chain may be further chemically modified; for example, galactose (Gal) and sialic acid (Sia) may be attached to the end.

[Formula 1]

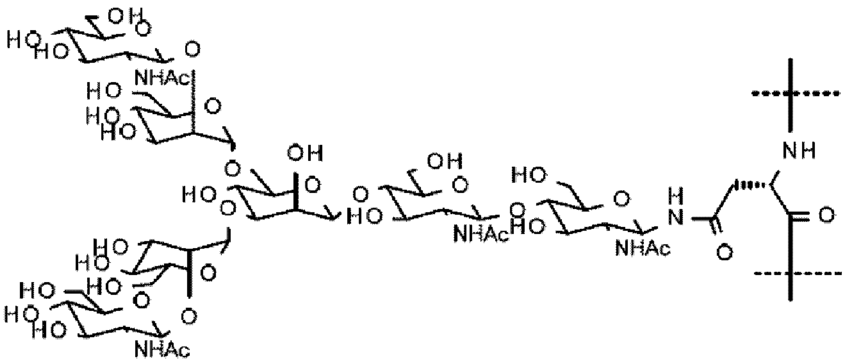

(I)

[Formula 2]

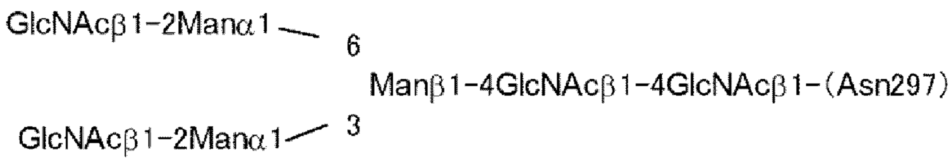

(II)

Most of the N297-linked sugar chains of IgG produced by cells have diversity of the sugar chain structure, including those with a sugar chain further linked to, e.g., a reducing end GlcNAc (core GlcNAc), a non-reducing end and/or a branched-chain sugar in the above basic structure. To the 6-position of core GlcNAc, fucose (Fuc) may be linked via $\alpha$-1,6 bond to form a modified structure having core fucose ((Fuc$\alpha$1,6)GlcNAc). In the case of Man which is a branched sugar, sometimes a sugar chain containing GlcNAc is linked to the 5-position thereof to form a triantennary sugar chain. To GlcNAc of the non-reducing end, a sugar chain containing galactose and sialic acid may sometimes be linked.

In the present invention, sialyl glycan (hereinafter referred to as "SG") has a basic structure represented by the following structural formula and array expression.

A typical example of SG is sialyl glycopeptide (hereinafter referred to as "SGP"), which is a sugar chain contained in chicken egg yolk.

[Formula 3]

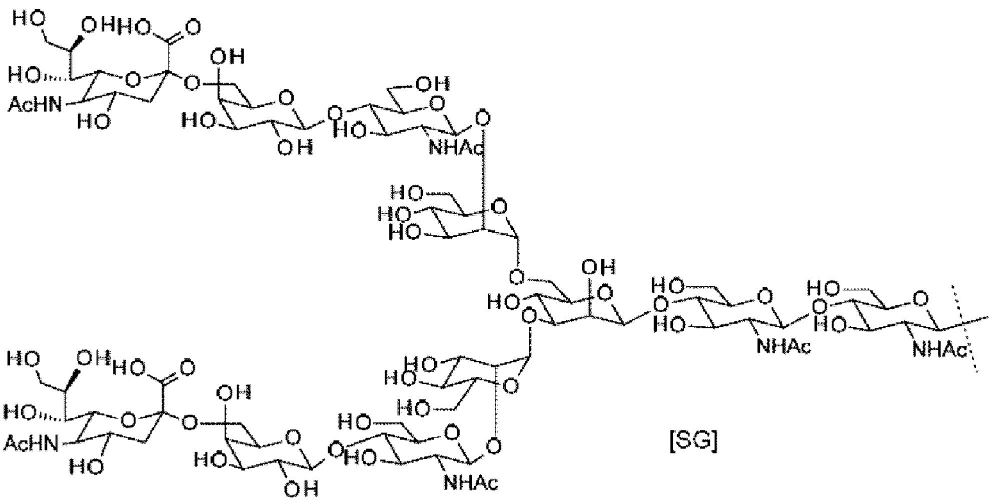

[Formula 4]

NeuAcα2-6Galβ1-4GlcNAcβ1-2Manα1— 6
Manβ1-4GlcNAcβ1-4GlcNAcβ1-(N/Q)
NeuAcα2-6Galβ1-4GlcNAcβ1-2Manα1— 3
[SG]

wherein "-(N/Q)" means binding to a side chain of Asn or Gln through N glycosidic bond.

For example, disialooctasaccharide (manufactured by Tokyo Chemical Industry Co., Ltd.), which consists only of a sugar chain (hereinafter referred to as "SG(10)") having a single deletion of GlcNAc in the reducing end of the sugar chain part of SG, is commercially available. In the description, the sugar chain structure having a sialic acid deletion at a non-reducing end only in one of the branched chains of β-mannose (β-Man) of SG(10) is referred to as MSG(9). The sugar chain structure having a sialic acid only at a 1-3 sugar chain of the branched chains is expressed as MSG1(9). The sugar chain structure having a sialic acid only at a 1-6 sugar chain of the branched chains is expressed as MSG2(9) (Patent Literature 1, WO2019/065964).

In the present invention, the "sugar chain donor molecule" refers to a sugar chain-containing molecule having activated GlcNAc at the reducing end, preferably oxazolylated GlcNAc, wherein molecules having a wide variety of sugar chain structures can be used. The "activated" state refers to the state where the reactivity of a sugar anomeric position is enhanced, including an oxazolylated state or a halogenated state. Examples of the sugar-chain donor molecule include [$N_3$-PEG(3)]-MSG1(9)-Ox (Figure 1) used in Example 6, SG(9)-Ox (oxazoline), MSG1(9)-Ox, MSG2(9)-Ox or a mixture of MSG1(9)-Ox and MSG2(9)-Ox.

Another aspect of the sugar-chain donor molecule is a sugar chain-containing molecule with non-activated GlcNAc at a reducing end, preferably, SGP (Figure 2), (SG-)Asn, (MSG1-)Asn, (MSG2-)Asn or a mixture of (MSG1-)Asn and (MSG2-)Asn.

In the present invention, unless otherwise specified, when a sugar chain links to a side chain of an amino acid, a partial structure thereof is expressed as, for example, "(SG-)Asn" where side chain moiety is placed in parentheses.

A sugar-chain donor molecule may be chemically modified. Examples of the sugar-chain donor molecule include sugar chain donor molecules having a non-reducing end chemical modified such as SGP, (SG-)Asn, (MSG1-)Asn, (MSG2-)Asn, a mixture of (MSG1-)Asn and (MSG2-)Asn, SG(10)-Ox, MSG1(9)-Ox, MSG2(9)-Ox or a mixture of MSG1(9)-Ox and MSG2(9)-Ox. Preferably, ([$N_3$-PEG(3)]$_2$-SG(10))-Ox, [$N_3$-PEG(3)]-MSG1(9)-Ox, [$N_3$-PEG(3)]-MSG2(9)-Ox or a mixture of [$N_3$-PEG(3)]-MSG1(9)-Ox and [$N_3$-PEG(3)]-MSG2(9)-Ox, or ([$N_3$-PEG(3)]$_2$-SG-)Asn-PEG(3)-$N_3$, ([$N_3$-PEG(3)]-MSG1-)Asn-PEG(3)-$N_3$, ([$N_3$-PEG(3)]-MSG2-)Asn-PEG(3)-$N_3$ or a mixture of ([$N_3$-PEG(3)]-MSG1-)Asn-PEG(3)-$N_3$ and ([$N_3$-PEG(3)]-MSG2-)Asn-PEG(3)-$N_3$ can be used (Patent Literature 1, WO2019/065964).

In the sugar-chain remodeling for drug discovery, it is preferable to employ a sugar chain donor having a humanized sugar chain or a human compatible sugar chain, which may not produce a significant problem when applied

to humans. Such a sugar chain is known to be non antigenic within a human body. Of N-linked sugar chains, for example, high mannose-type, hybrid-type and complex-type sugar chains are known. These three sugar chains are common in basic structure. The high mannose-type sugar chain has a mannose rich structure where a plurality of mannose molecules are continuously present in the two branched chains (1-3 chain, 1-6 chain), which are branched from mannose (β-mannose) located close to the reducing end. The hybrid-type sugar chain has a structure having GlcNAc in one of the two branched chains (1-3 chain, 1-6 chain), which are branched from mannose (β-mannose) located close to the reducing end. The complex-type sugar chain has a structure having GlcNAc in the two branched chains (1-3 chain, 1-6 chain), which are branched from mannose (β-mannose) located close to the reducing end. The structure is diversified depending on the presence or absence of galactose, the presence or absence of sial and linkage isomerization/positional isomerization of these. As the complex-type sugar chain, biantennary type, triantennary type and quadantennary type sugar chains are known.

Examples of the structures of high mannose-type, hybrid-type and complex-type sugar chains will be shown below.

[Formula 5]

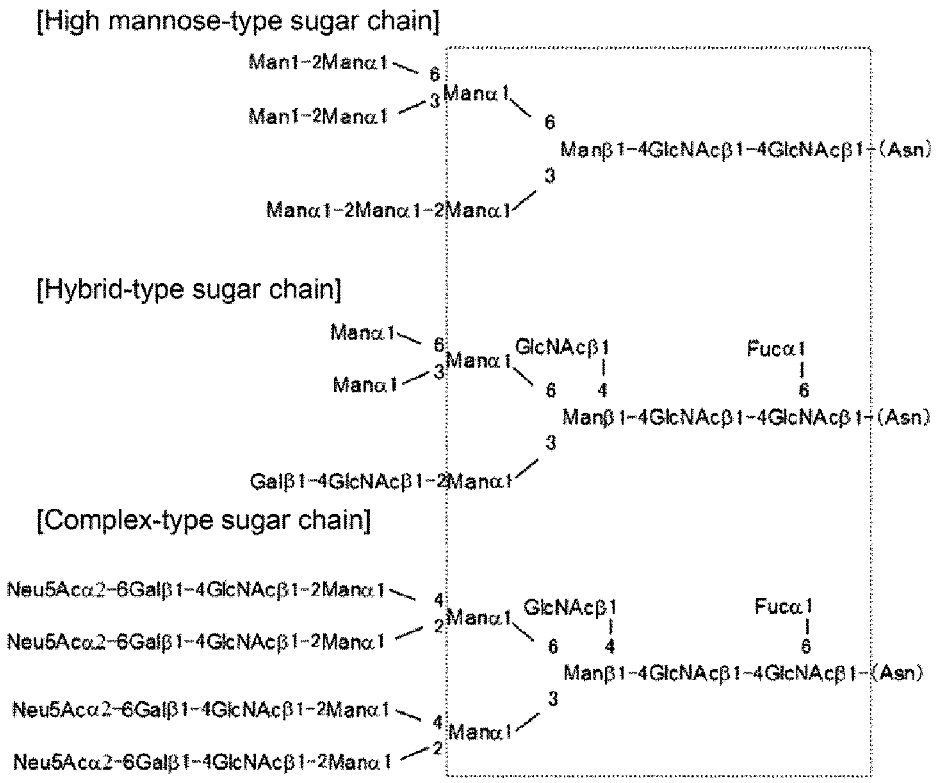
[High mannose-type sugar chain]
[Hybrid-type sugar chain]
[Complex-type sugar chain]

Types of humanized N-linked sugar chains

In the present invention, the "acceptor molecule" refers to a molecule containing a sugar structure having GlcNAc at a non-reducing end. When the "acceptor molecule" is reacted with a sugar-chain donor molecule in the presence of Endo-Si or a mutant Endo-Si, an oxazoline ring of the sugar-chain donor molecule or an active intermediate (Non-Patent Literature 9) reacts to the 4-position of GlcNAc of the non-reducing end to form a chitobiose structure.

A typical acceptor molecule is IgG or the Fc fragment of IgG, which is derived from a monoclonal antibody and having an N297-linked sugar chain only consisting of core GlcNAc optionally having core Fuc linked. To the core GlcNAc, core Fuc may or may not bind depending of its original antibody or a production method thereof. As the origin of the acceptor molecule, various monoclonal antibodies or sugar chain-containing molecules or Fc region-containing molecules (e.g., Fc, CLCH formed by combining CH which consists only of a constant region obtained by removing a variable region from a heavy chain, and CL which consists only of a constant region of a light chain) can be used. Preferably, (Fucα1, 6)-GlcNAc-IgG (for example, (Fucα1, 6) GlcNAc-mAb1 shown in Figure 4), (Fucα1, 6)-GlcNAc-Fc, and (Fucα1, 6)-GlcNAc-CLCH (Patent Literature 1), can be used.

In the present invention, the "Endo-Si" represents a kind of an endo-β-N-acetylglucosaminidase (ENGase)

derived from *Streptococcus iniae.* The nucleotide sequence thereof is represented by SEQ ID NO: 1 and the amino acid sequence thereof is represented by SEQ ID NO: 2. Endo-Si is an enzyme consisting of the amino acid sequence at amino acid positions 34 to 928 in SEQ ID NO: 2 (amino acids at positions 1 to 33 represent a signal sequence. The signal sequence was estimated by a tool, "SignalP-5.0" provided by CBS) in which the amino acid at position 241 is Asp (EC 3.2.1. 96, GH18). Endo-Si specifically recognizes an N-linked sugar chain (for example, an N297-linked sugar chain) and has both hydrolysis activity and transglycosylation activity.

The hydrolysis activity of Endo-Si refers to an activity to specifically hydrolyze a β-1, 4 glycosidic bond contained in core chitobiose of an N-linked sugar chain having a basic structure as mentioned above (in the description unless otherwise specified, "hydrolysis activity" refers to the activity defined above. The schematic illustration of the (hydrolysis) reaction is shown in Figure 4).

The transglycosylation activity of Endo-Si refers to an activity to bind a reducing end of a sugar-chain donor molecule as mentioned above (sugar chain-containing molecule having GlcNAc having a reducing end activated or not activated) to an acceptor molecule containing a Fc site having only core GlcNAc (core fucose may or may not be added) at N297, via a glycosidic bond (hereinafter the activity will be referred to as "transglycosylation activity, the schematic illustration of the reaction is shown in Figure 5 or Figure 17).

The substrate specificities of Endo-Si to various antibodies are as follows. Endo-Si exhibits sugar chain hydrolysis activity on all of 4 subclasses of IgG but does not exhibit sugar chain hydrolysis activity on IgA and IgE. The substrate specificities of Endo-Si to various N-linked sugar chains are as follows. Endo-Si exhibits hydrolysis activity on high mannose-type sugar chain and complex-type biantennary sugar chain, but the specificity to the complex-type biantennary sugar chain is higher than that of the high mannose-type sugar chain. Of them, the hydrolysis activity on a G0 sugar chain is the highest. Endo-Si further exhibits hydrolysis activity on a sialyl sugar chain and a fucosylated sugar chain but does not exhibit hydrolysis activity on a complex triantennary sugar chain.

Furthermore, the G0 sugar chain is GlcNAc, in which non-reducing ends bind to two branched chains (1-3 chain, 1-6 chain), in other words, a biantennary complex-type sugar chain having no galactose residues.

The enzyme of the present invention is not limited to the enzyme having a predetermined sequence obtained in Examples as long as it has the above characteristics, and may be an enzyme isolated from a natural source or artificially prepared, or modified based on the sequence information of the enzyme of the present invention. In the case of an enzyme isolated from a natural source, a biological species used as the source is not particularly limited but it is preferably a bacterium, more preferably a bacterium of the genus *Streptococcus*, and further preferably a bacterium belonging to *Streptococcus iniae.*

Endo-Si active domain and a carbohydrate-binding module (CBM) are estimated as a region at amino acid positions 106 to 447 and a region at amino acid positions 762 to 897 in SEQ ID NO: 2, respectively, based on comparison with the sequence of EndoS whose crystal structure has been analyzed (B. Trastoy et al., PNAS (2014) vol, 111, No. 18, pp 6714-6719). These two regions are considered as important positions for interaction between hydrolysis activity and/or transglycosylation activity and an antibody. Thus, as the enzyme of the present invention, a polypeptide containing an amino acid sequence at amino acid positions 106 to 447 and/or amino acid positions 762 to 897 in SEQ ID NO: 2, preferably, an amino acid sequence at amino acid positions 106 to 897 in SEQ ID NO: 2, more preferably an amino acid sequence at amino acid positions 106 to 928 in SEQ ID NO: 2; and further preferably an amino acid sequence at amino acid positions 34 to 928 in SEQ ID NO: 2; and exhibiting hydrolysis activity and/or transglycosylation activity.

<Mutant enzyme of the present invention>

The present invention provides an Endo-Si mutant enzyme, characterized by having the amino acid sequence at amino acid positions 34 to 928 in SEQ ID NO: 2, the amino acid sequence having the same amino acid sequence except containing one or more mutations at one or more positions amino acid selected from the group consisting of amino acids at position 241 (D241), position 190 (T190), position 311 (Q311) and position 360 (E360), polypeptide exhibiting sugar chain hydrolysis activity and/or transglycosylation activity; and preferably ,as shown in Example 6 provides an Endo-Si mutant enzyme characterized by containing a region necessary for transglycosylation activity in the amino acid sequence at amino acid positions 34 to 928 in SEQ ID NO: 2, and having a reduced hydrolysis activity and an improved transglycosylation activity, which are the activities on the N297-linked sugar chain of IgG, compared to the activity Endo-Si WT (hereinafter, a wild strain having no mutation introduced in the amino acid sequence will be referred to "WT").

The substitution/mutation of an amino acid of the present invention is that having the aforementioned characteristics, preferably substitution/mutation at least one or more amino acid positions selected from T190, D241, Q311 and E360 in SEQ ID NO: 2, more preferably, D241Q, D241M, D241A, T190Q, Q311L, E360Q, E360A, E360N or E360D, further preferably, D241Q, D241M, T190Q, Q311L and/or E360Q and most preferably, D241Q, D241Q/Q311L, D241Q/E360Q, D241M, D241M/Q311L, D241M/E360Q, T190Q/D241Q, T190Q and/or T190Q/D241M. Furthermore, as long as the substitution/mutation has the aforementioned characteristics, another substitution/mutation may be contained in addition to the substitutions/mutations at T190, D241, Q311 and/or E360 in SEQ ID NO: 2.

Endo-β-N-acetylglucosaminidase has both hydrolysis activity and transglycosylation activity (hereinafter both activities will be referred to as the "enzymatic activities". Because of this, an enzyme retaining a strong hydrolysis activity hydrolyzes a sugar chain, which was previously transferred to core GlcNAc of an acceptor molecule (an antibody or its Fc region-containing molecule having core GlcNAc as the N297-linked sugar chain) by transglycosylation activity, as a substrate. As the result, a desirable transglycosylated molecule may not be obtained appropriately. For this reason, in synthesizing, such as, a sugar chain remodeling antibody or a sugar chain modified compound, a mutant enzyme improved in transglycosylation activity is useful. The mutant enzyme of the present invention is characterized by having both reducing hydrolysis activity and enhancing transglycosylation activity than Endo-Si WT.

The transglycosylation activity of a mutant enzyme can be evaluated by the method described later in Example 6 or Example 7.

Of the enzymatic activities that the mutant enzyme of the present invention has, the transglycosylation activity is higher than that of Endo-Si WT. In other words, a sugar chain donor (for example, a sugar chain containing GlcNac activated at the reducing end (e.g., GlcNAc oxazolylated)) exhibits a higher transglycosylation rate than that of Endo-Si WT at pH 7 to 8 (for example, pH 7.5), in the conditions where an acceptor molecule is present in an amount of 5 to 10 equivalents (for example, 8 equivalents), on and after any time of 1 to 24 or 48 hours after initiation of the reaction. Preferably, the transglycosylation rate exceeds 50% up to 24 hours or 48 hours after initiation of the reaction, more preferably, the transglycosylation rate exceeds 60% up to 24 hours after initiation of the reaction, further preferably, the transglycosylation rate exceeds 80% up to 24 hours after initiation of the reaction, and even more preferably, the transglycosylation rate exceeds 95% up to 24 hours after initiation of the reaction.

As another aspect, the transglycosylation activity that the mutant enzyme of the present invention has exceeds that of Endo-Si WT. In other words, a sugar chain donor containing non-activated GlcNac at a reducing end, exhibits the same or higher transglycosylation rate than that of Endo-Si WT at pH 7 to 8 (for example, pH 7.5), in the conditions where an acceptor molecule is present in an amount of 10 to 100 equivalents (for example, 50 equivalents) on and after any time of 1 to 48 hours after initiation of the reaction. Preferably, the transglycosylation rate exceeds 50% up to 24 hours or 48 hours after initiation of the reaction, more preferably, the transglycosylation rate exceeds 60% up to 48 hours after initiation of the reaction, further preferably, the transglycosylation rate exceeds 80% up to 48 hours after initiation of the reaction, and even more preferably, the transglycosylation rate exceeds 90% up to 48 hours after initiation of the reaction.

The mutant enzyme of the present invention may not always have a full length, as long as it has one or more mutations (preferably 1 to 3, and more preferably 1 or 2) at one or more amino acid positions selected from the group consisting of amino acids at position D241, T190, Q311 and E360 in the amino acid sequence at amino acid positions 34 to 928 in SEQ ID NO: 2, and as long as it has a region important for transglycosylation activity of Endo-Si. Domain analysis of EndoS revealed that a catalytic domain (at amino acid positions 106 to 447 in SEQ ID NO: 2), and/or CBM (at amino acid positions 762 to 897 in SEQ ID NO: 2) are important sites. As long as an enzyme contains these sites, the enzyme can be used as the mutant enzyme of the present invention.

The mutant enzyme of the present invention is a polypeptide containing a mutation described in the above section "Substitution/mutation of an amino acid of the present invention"; more specifically, a polypeptide containing an amino acid sequence selected from the group consisting of SEQ ID NO: 3 (Endo-Si D241Q), SEQ ID NO: 4 (Endo-Si D241Q/Q311L), SEQ ID NO: 5 (Endo-Si D241Q/E360Q), SEQ ID NO: 6 (Endo-Si D241M), SEQ ID NO: 7 (Endo-Si D241M/Q311L), SEQ ID NO: 8 (Endo-Si D241M/E360Q), SEQ ID NO: 9 (Endo-Si T190Q/D241Q), SEQ ID NO: 10 (Endo-Si T190Q) and SEQ ID NO: 11 (Endo-Si T190Q/D241M).

In amino acid sequence of mutant enzyme of the present invention, substitution, deletion, insertion, and/or addition of one to several amino acids may be present at the positions excluding the essential mutation at position (D241, T190, Q311 or E360) as long as it does not affect the enzymatic activities. As the amino acid mutation site, the all positions may be selected as long as the enzymatic activities are not affected; preferably amino acids excluding amino acids at positions 241, 190, 311 or 360, more preferably positions excluding a catalytic domain (at amino acid positions 106 to 447 in SEQ ID NO: 2) and CBM (at amino acid positions 762 to 897 in SEQ ID NO: 2), and further preferably positions contained in the region at amino acid positions No. 34 to 105 or amino acid positions 898 to 928 in SEQ ID NO: 2.

In the present invention, the "several" refers to 30 or 20 or less, preferably 10 or less, further preferably 5 or less, and most preferably 4, 3, 2 or 1.

In the present invention, the amino acid after substitution by mutation is not particularly limited as long as the mutant enzyme finally obtained has the enzymatic activities. Various amino acids can be employed, such as a naturally occurring amino acid, an artificially synthesized amino acid and modified amino acids. Preferably a naturally occurring amino acid, more preferably a naturally occurring L-amino acid, and further preferably an essential amino acid.

The amino acid sequence of mutant enzyme of the present invention has a homology or identity of at least 80% or more, preferably 85% or more, more preferably 90% or more, further preferably 95%, 96%, 97%, 98% or 99% or more to each of the amino acid sequences excluding an amino acid of the essential mutation (D241, T190, Q311 or

E360) as long as the enzymatic activities are not affected.

The identity or homology between two amino acid sequences can be determined by use of default parameters, Blast algorithm version 2. 2. 2 (Altschul, SF, et al., Nucleic Acids Res. 1997, 25, 3389-3402). Blast algorithm is available by accessing an Internet Web site, for example, http://blast. ncbi. nlm. nih. gov/.

<Gene, host cell, enzyme production method>

The present invention further provides, e.g., a recombinant gene encoding Endo-Si (SEQ ID NO: 1) or an Endo-Si mutant enzyme as mentioned above; a gene construct such as a plasmid and expression vector containing the recombinant gene; a host cell transformed with the gene construct; and a method for producing the enzyme of the present invention including a step of recovering an Endo-Si or an Endo-Si mutant enzyme of the present invention from a culture of the host cell. The recombinant gene, gene construct, host cell and others can be prepared in accordance with a genetic engineering method commonly known in the technical field based on the amino acid sequence of the mutant enzyme of the present invention. The nucleotide sequence of Endo-Si for *Escherichia coli* is represented by SEQ ID NO: 16.

A host cell (appropriately selected from cells usually used for producing a protein, such as animal cells, plant cells, *Escherichia coli* and yeasts) transformed with introduction of a gene encoding the enzyme of the present invention, is cultured in appropriate conditions depending on the type of cell. From the culture product, the enzyme of the present invention can be recovered. The enzyme is recovered based on the physical properties of the enzyme by using purification methods routinely used, appropriately in combination. To simplify the recovery, a gene construct is previously designed by ligating a tag peptide, such as His tag and GST tag, to the enzyme, so that recovery can be made by use of affinity of the tag peptide. The tag peptide may be removed after purification. If a tag peptide does not affect enzyme activity, the enzyme attached with the tag peptide may be subjected to a reaction such as a sugar chain remodeling reaction. The enzyme of the present invention includes an enzyme having an amino acid sequence including the amino acid sequence of a tag peptide.

<Sugar chain remodeling>

The present invention provides a method for remodeling a sugar chain of a glycoprotein using the Endo-Si or an Endo-Si mutant enzyme of the present invention and a glycoprotein having a sugar chain consisting of a substantially homogeneous structure produced by the sugar chain remodeling. In addition, the present invention also provides a method for producing a glycoprotein having a sugar chain consisting of a substantially homogeneous structure by the sugar chain remodeling.

According to an aspect of the present invention, the present invention provides a method for remodeling an N-linked sugar chain containing an N297 link in an antibody or its Fc region-containing molecule by use of the Endo-Si or an Endo-Si mutant enzyme of the present invention, and a glycoprotein, preferably an antibody or an Fc region- containing molecule, having an N-linked sugar chain containing an N297 link consisting of substantially homogeneous structure produced by the sugar chain remodeling. The present invention further provides a method for producing a glycoprotein, preferably an antibody or an Fc region-containing molecule, having an N-linked sugar chain containing an N297 link consisting of substantially homogeneous structure produced by the sugar chain remodeling. The antibody is preferably IgG. IgG will be described below. In the present invention, the glycoprotein is a protein, which is present in animal/plant tissues, and cell membranes and cell walls of eukaryotic microorganisms, having at least one O-linked sugar chain or N-linked sugar chains bound within the amino acid sequence thereof. The glycoprotein may be derived from a natural source or synthesized and refers to, for example, a monoclonal antibody IgG, or an Fc region-containing molecule such as an Fc fragment of IgG or CLCH consisting only of a constant region of IgG (Patent Literature 1, WO2018/003983).

The "sugar chain remodeling" refers to a method for producing IgG or its Fc region containing molecule having an N297-linked sugar chain derived from a sugar chain donor and having a homogeneous sugar chain structure, first by preparing an acceptor molecule by cutting away the N297-linked sugar chain of a predetermined glycoprotein, e.g., a monoclonal antibody IgG, or an Fc region-containing molecule such as an Fc fragment of IgG or CLCH consisting only of a constant region of IgG while core GlcNAc (optionally core fucose is added) is allowed to remain intact, and next by transferring a sugar chain derived from a sugar chain donor to the core GlcNAc of the acceptor molecule by use of the transglycosylation activity of the Endo-Si mutant enzyme of the present invention.

The IgG or an Fc region-containing molecule for use in remodeling a sugar chain is not limited as long as they are derived preferably from the IgG heavy chain consisting of the same amino acid sequence and they are produced so as to contain an N297-linked sugar chain. A method for producing IgG or an Fc region-containing molecule is not limited. IgG produced by a commonly known method for producing a monoclonal antibody, CLCH of IgG or an Fc fragment obtained by enzymatically treating IgG or CLCH, can be used. Such IgG or an Fc fragment may also employ a mixture of samples obtained by different production methods or in different lots.

An acceptor molecule for use in remodeling a sugar chain can be prepared by a method of treating IgG or an Fc region-containing molecule (as mentioned above) with ENGase having an activity to specifically hydrolyze a 1,4-glycosidic bond (GlcNAc β-1-4GlcNAc) between GlcNAc molecules in core chitobiose structure of an N297-linked sugar chain. Examples of the ENGase employed herein include various enzymes such as Endo-Si WT, Endo-A, Endo-D, Endo-E, Endo-F3, Endo-H, EndoS and EndoS2.

As the sugar-chain donor molecule for use in sugar chain remodeling, sugar-chain donor molecules different in sugar chain structure can be employed. For the purpose of using the antibody remodeled as an antibody drug, a sugar chain donor having a sugar chain structure analogous or identical to that of a human, or humanized or human-compatible sugar chain structure, is preferably employed.

As a typical sugar-chain donor molecule, a molecule having an N-linked sugar chain as mentioned above as the basic structure and prepared by removing core GlcNAc and activating the second GlcNAc from the reducing end, can be mentioned. For example, SG(10)-Ox, ([$N_3$-PEG(3)]$_2$-SG(10))-Ox, [$N_3$-PEG(3)]-MSG1(9)-Ox, [$N_3$-PEG(3)]-MSG2(9)-Ox or a mixture of [$N_3$-PEG(3)]-MSG1(9)-Ox and [$N_3$-PEG(3)]-MSG2(9)-Ox, can be used (WO2019/065964).

As another aspect of the sugar-chain donor molecule, a molecule in which the second GlcNAc from the reducing end is not activated can be used. For example, ([$N_3$-PEG(3)]$_2$-SG-)Asn-PEG(3)-$N_3$, ([$N_3$-PEG(3)]-MSG1-)Asn-PEG(3)-$N_3$, ([$N_3$-PEG(3)]-MSG2-)Asn-PEG(3)-$N_3$ or a mixture of ([$N_3$-PEG(3)]-MSG1-)Asn-PEG(3)-$N_3$ and ([$N_3$-PEG(3)]-MSG2-)Asn-PEG(3)-$N_3$ can be used (Patent Literature 1, WO2019/065964).

At this time, in the presence of both endo-β-N-acetylglucosaminidase (Enzyme A) that prefers a complex-type sugar chain of a sugar-chain donor molecule as a substrate but does not prefer an N297-linked sugar chain as a substrate, and the Endo-Si or an Endo-Si mutant enzyme of the present invention, preferably an Endo-Si mutant enzyme, a sugar chain of a donor molecule is linked to a core GlcNAc residue of IgG or an Fc region-containing molecule, which is obtained by cutting away a sugar chain and serves as an acceptor molecule. If Enzyme A used herein has transglycosylation activity from SGP serving as a sugar chain donor to an acceptor (molecule) having GlcNAc, Enzyme A exhibits a high transglycosylation efficiency in a one-pot method. In other words, Enzyme A can be selected from endo-β-N-acetylglucosaminidases, which prefers a complex-type sugar chain of a sugar-chain donor molecule having a reducing end not activated as a substrate but does not prefer an N297-linked sugar chain as a substrate, based on the transglycosylation activity on an acceptor (molecule) having GlcNAc, as an index. The endo-β-N-acetylglucosaminidase, which does not prefer an N297-linked sugar chain as a substrate include Endo-M, Endo-Rp, Endo-Om, Endo-CC and mutant enzymes thereof having a reduced hydrolysis activity. Mutant enzymes with reduced hydrolysis activity include commonly known Endo-Rp N172Q, Endo-Rp N172H (Patent Literature 3), Endo-M N175Q (Umekawa M. et al., J Biol Chem. 2010, 285, 511-521), Endo-CC N180H or Endo-Om N194Q (Chiba Y. Kagaku to Seibutsu 2015, 53, 236-244). Preferably, Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T and Endo-Rp N172V. In addition, examples of a mutant enzyme obtained by substituting two amino acids include Endo-Rp W278F/S216V, Endo-Rp W278F/N246D, Endo-Rp W278F/D276N and Endo-Rp W278F/A310D. Furthermore, examples of a mutant enzyme obtained by substituting three amino acids include Endo-Rp W278F/N172D/F307Y, Endo-Rp W278F/N172D/F307H, Endo-Rp W278F/N172D/A310D and Endo-Rp W214FIF307YIL306I.

The amino acid sequences of Endo-Rp mutant enzymes are represented by SEQ ID NOs: 17 to 43. The amino acid sequence of Endo-M is represented by SEQ ID NO: 44. The amino acid sequence of Endo-Om is represented by SEQ ID NO: 45. The amino acid sequence of Endo-CC is represented by SEQ ID NO: 46.

Examples of a combination of an Endo-Si mutant enzyme of the present invention and Enzyme A are shown in Table 1 and Table 2.

[Table 1]

| Endo-Si mutant enzyme | Enzyme A (Endo-Rp mutant enzyme) |
|---|---|
| D241Q | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214FIF307Y/L306I |

(continued)

| Endo-Si mutant enzyme | Enzyme A (Endo-Rp mutant enzyme) |
|---|---|
| D241M | N172Q |
| | N172H |
| | N172A |
| | N172C |
| | N172D |
| | N172E |
| | N172G |
| | N1721 |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| D241A | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| T190Q | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| Q311L | N172Q |
| | N172H |
| | N172A |
| | N172C |
| | N172D |
| | N172E |
| | N172G |
| | N1721 |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| E360Q | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| E360A | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| E360N | N172Q |
| | N172H |
| | N172A |
| | N172C |
| | N172D |
| | N172E |
| | N172G |
| | N1721 |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| E360D | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| D241Q/Q311L | N172Q |
| | N172H |
| | N172A |
| | N172C |
| | N172D |
| | N172E |
| | N172G |
| | N1721 |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| D241Q/E360Q | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278FIN172DIF307H |
| | W278FIN172DIA310D |
| | W214F/F307Y/L306I |

(continued)

| Endo-Si mutant enzyme | Enzyme A (Endo-Rp mutant enzyme) |
|---|---|
| D241M/Q311L | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

(continued)

| Endo-Si mutant enzyme | Enzyme A (Endo-Rp mutant enzyme) |
|---|---|
| D241M/E360Q | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278FIN172DIF307H |
| | W278FIN172DIA310D |
| | W214F/F307Y/L306I |

(continued)

| Endo-Si mutant enzyme | Enzyme A (Endo-Rp mutant enzyme) |
|---|---|
| T190Q/D241Q | N172Q |
| | N172H |
| | N172A |
| | N172C |
| | N172D |
| | N172E |
| | N172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278FIN172DIF307H |
| | W278FIN172DIA310D |
| | W214F/F307Y/L306I |

(continued)

| Endo-Si mutant enzyme | Enzyme A (Endo-Rp mutant enzyme) |
|---|---|
| T190Q/D241M | N 172Q |
| | N172H |
| | N172A |
| | N172C |
| | N172D |
| | N172E |
| | N 172G |
| | N172I |
| | N172L |
| | N172M |
| | N172P |
| | N172S |
| | N172T |
| | N172V |
| | W278F/S216V |
| | W278F/N246D |
| | W278F/D276N |
| | W278F/A310D |
| | W278F/N172D/F307Y |
| | W278F/N172D/F307H |
| | W278F/N172D/A310D |
| | W214F/F307Y/L306I |

[Table 2]

| Endo-Si mutant enzyme | Enzyme A (other mutant enzymes) |
|---|---|
| D241Q | Endo-M N175Q |
| D241M | |
| D241A | |
| T190Q | |
| Q311L | |
| E360Q | |
| E360A | |
| E360N | |
| E360D | |
| D241Q/Q311L | |
| D241Q/E360Q | |
| D241M/Q311L | |
| D241M/E360Q | |
| T190Q/D241Q | |
| T190Q/D241M | |
| D241Q | Endo-CC N180H |
| D241M | |
| D241A | |
| T190Q | |
| Q311L | |
| E360Q | |
| E360A | |
| E360N | |
| E360D | |
| D241Q/Q311L | |
| D241Q/E360Q | |
| D241M/Q311L | |
| D241M/E360Q | |
| T190Q/D241Q | |
| T190Q/D241M | |

(continued)

| Endo-Si mutant enzyme | Enzyme A (other mutant enzymes) |
|---|---|
| D241Q | Endo-Om N194Q |
| D241M | |
| D241A | |
| T190Q | |
| Q311L | |
| E360Q | |
| E360A | |
| E360N | |
| E360D | |
| D241Q/Q311L | |
| D241Q/E360Q | |
| D241M/Q311L | |
| D241M/E360Q | |
| T190Q/D241Q | |
| T190Q/D241M | |

Examples of a preferable combination of the Endo-Si mutant enzyme of the present invention and Enzyme A for producing a glycoprotein having a sugar chain consisting of a substantially homogeneous structure produced by sugar chain remodeling, are shown in Table 3.

[Table 3]

| Endo-Si mutant enzyme | Enzyme A |
|---|---|
| D241Q | Endo-Rp N172H |
| D241M | |
| D241A | |
| T190Q | |
| Q311L | |
| E360Q | |
| E360A | |
| E360N | |
| E360D | |
| D241Q/Q311L | |
| D241Q/E360Q | |
| D241M/Q311L | |
| D241M/E360Q | |
| T190Q/D241Q | |
| T190Q/D241M | |

(continued)

| Endo-Si mutant enzyme | Enzyme A |
|---|---|
| D241Q | Endo-M N175Q |
| D241M | |
| T190Q | |
| D241Q/Q311L | |
| D241Q/E360Q | |
| D241M/Q311L | |
| D241 M/E360Q | |
| T190Q/D241Q | |
| T190Q/D241M | |

In the presence of a polypeptide of the present invention exhibiting transglycosylation activity and endo-β-N-acetylglucosaminidase (Enzyme A), which prefers a complex-type sugar chain of a sugar-chain donor molecule having a reducing end not activated as a substrate but does not prefer an N297-linked sugar chain as a substrate, an acceptor molecule, which is an antibody or its Fc region-containing molecule, having, as an N297-linked sugar chain, core GlcNAc optionally having fucose added thereto, may be reacted with a sugar chain donor molecule containing a sugar chain having a reducing end not activated (Figure 17).

The reaction conditions of hydrolysis reaction for preparing an acceptor molecule in remodeling a sugar chain can be appropriately selected in accordance with conditions for other enzymes known in the technical field in consideration of, e.g., the enzyme activity, the property of an antibody, the recovery rate in a purification step and working hours. The reaction is carried out in a buffer, which can be appropriately selected from buffers commonly used for enzyme reactions, such as a citrate buffer (pH 3.5 to 5.5), an acetate buffer (pH 4.5 to 6.0), a phosphate buffer (pH 6.0 to 7.5), a MOPS-NaOH buffer (pH 6.5 to 8.0) and a Tris-HCl buffer (pH 7.0 to 9.0). Preferably, a phosphate buffer (pH 6.0 to 7.5) or a Tris-HCl buffer (pH 7.0 to 9.0) is used. To a reaction solution, additives, which do not inhibit an enzymatic reaction, may be added in order to stabilize the enzyme, but the additives may not be added.

The reaction temperature can be appropriately selected in the range of 4°C to 50°C, and is preferably 15°C to 45°C, more preferably 18°C to 40°C, and more preferably 20°C to 35°C.

The reaction pH of hydrolysis reaction by Endo-Si can be appropriately selected in the range of pH 5.8 to 9.5, and is preferably, pH 6.2 to pH 8.0, and more preferably pH 6.5 to pH 7.5.

The reaction time can be appropriately selected in the range of 10 minutes to 96 hours, and is preferably, 0.5 hours to 80 hours, more preferably, 1 hour to 60 hours, more preferably 8 hours to 48 hours, and more preferably 12 to 24 hours. The completion of the reaction can be determined by taking a small amount of the reaction solution with time and checking the degree of progress of the hydrolysis. In general, the degree of progress of the sugar chain hydrolysis reaction can be monitored by, e.g., sodium dodecyl sulfate acid -polyacrylamide gel electrophoresis (SDS-PAGE), a fully automated electrophoresis system or liquid chromatography mass spectrometry (LC-MS). In this patent, after the degree of progress of the sugar chain hydrolysis reaction was checked by fragmenting a commercially available antibody or a sugar chain remodeling antibody into a heavy chain and a light chain, and confirming that the retention time only on the side of the heavy chain fragment, to which the N297-linked sugar chain was attached, changes, by use of a fully automated electrophoresis system.

The transglycosylation reaction conditions using GlcNAc (e.g., GlcNAc oxazolylated) having a reducing end activated as a sugar chain donor or GlcNAc having a reducing end not activated as a sugar chain donor in sugar chain remodeling, can be appropriately selected in accordance with conditions commonly known in other enzyme cases (e.g., Patent Literature 1, WO2019/065964).

The reaction is carried out in a buffer. One that does not promote decomposition of a sugar chain donor containing GlcNAc having a reducing end activated or not activated, is desirably used. The buffer can be appropriately selected from, e.g., a phosphate buffer (pH 6.0 to 7.5), an MOPS-NaOH buffer (pH 6.5 to 8.0) and a Tris-HCl buffer (pH 7.0 to 9.0) and is preferably a Tris-HCl buffer (pH 7.0 to 9.0). Additives that do not inhibit an enzymatic reaction may be added to the reaction solution in order to stabilize an enzyme, but additives may not be added.

The reaction temperature can be appropriately selected in the range of 4°C to 50°C, and is 15°C to 45°C, more preferably 20°C to 40°C, and more preferably 25°C to 40°C.

The reaction pH of the hydrolysis reaction by Endo-Si can be appropriately selected in the range of pH 5.8 to

9.5, and is preferably, pH 6.2 to pH 8.0, and more preferably pH 6.5 to pH 7.5.

The reaction time can be appropriately selected in the range of 10 minutes to 96 hours, and is preferably, 0.5 hours to 80 hours, more preferably, 2 hours to 70 hours, more preferably 12 hours to 60 hours, more preferably 16 to 48 hours, and more preferably 16 to 28 hours. The completion of the reaction can be determined by taking a small amount of the reaction solution with time and checking the degree of progress of the transglycosylation reaction. In general, the degree of progress of the transglycosylation can be monitored by, e.g., sodium dodecyl sulfate acid - polyacrylamide gel electrophoresis (SDS-PAGE), a fully automated electrophoresis system or liquid chromatography mass spectrometry (LC-MS). In this patent, the degree of progress of the transglycosylation reaction was checked by fragmenting a commercially available antibody or a sugar chain remodeling antibody into a heavy chain and a light chain, and confirming that the retention time only on the side of the heavy chain fragment, to which the N297-linked sugar chain was attached, changes, by use of a fully automated electrophoresis system.

Remodeling of a sugar chain can be carried out by a method (one pot method) of directly transferring a sugar chain of a sugar-chain donor molecule to an acceptor molecule, i.e., an antibody or its Fc region-containing molecule having, as an N297-linked sugar chain, core GlcNAc optionally having fucose added thereto, by use of both ENGase, which has an activity to specifically hydrolyze 1,4-glycosidic bond (GlcNAcβ1-4GlcNAc) between GlcNAc molecules in a core chitobiose structure of an N297-linked sugar chain, and Enzyme A as mentioned above. As the ENGase having an activity to specifically hydrolyze 1,4-glycosidic bond (GlcNAcβ1-4GlcNAc) between GlcNAc molecules in the core chitobiose structure of an N297-linked sugar chain, an Endo-Si mutant enzyme as mentioned above is preferable. As Enzyme A, an Endo-Rp mutant enzyme as mentioned above lowered in hydrolysis activity is preferable. In the one-pot method, as the sugar-chain donor molecule, SGP, (SG-)Asn, (MSG1-)Asn, (MSG2-)Asn, a mixture of (MSG1-)Asn and (MSG2-)Asn, SG(10)-Ox, MSG1(9)-Ox, MSG2(9)-Ox or a mixture of MSG1(9)-Ox and MSG2(9)-Ox, which is a sugar-chain donor molecule having a non-reducing end chemically modified, is included. Preferably, e.g., ([$N_3$-PEG(3)]$_2$-SG(10))-Ox, [$N_3$-PEG(3)]-MSG1(9)-Ox, [$N_3$-PEG(3)]-MSG2(9)-Ox or a mixture of [$N_3$-PEG(3)]-MSG1(9)-Ox and [$N_3$-PEG(3)]-MSG2(9)-Ox or ([$N_3$-PEG(3)]$_2$-SG-)Asn-PEG(3)-$N_3$, ([$N_3$-PEG(3)]-MSG1-)Asn-PEG(3)-$N_3$, ([$N_3$-PEG(3)]-MSG2-)Asn-PEG(3)-$N_3$ or a mixture of ([$N_3$-PEG(3)]-MSG1-)Asn-PEG(3)-$N_3$ and ([$N_3$-PEG(3)]-MSG2-)Asn-PEG(3)-$N_3$ can be used (Patent Literature 1, WO2019/065964).

A glycoprotein (antibody or an Fc region-containing molecule) produced by the sugar chain remodeling method can be further chemically or biochemically modified. For example, an azide group ($N_3$-) is reacted with, a group having an alkyne structure such as a (hetero)cycloalkynyl group (for example, DBCO (dibenzocyclooctyne)) to form a 1,2,3-triazole ring (SPAAC (strain-promoted alkyne azide cycloaddition: Agard NJ, et al., J Am Chem Soc. 2004, 126, 46, 15046-15047)). Accordingly, the sugar chain remodeling antibody obtained by use of a donor molecule having an azide group ($N_3$-) as mentioned above is reacted with a molecule having a (hetero)cycloalkynyl group and a desired activity (pharmaceutical active compound (for example, a chemotherapeutic agent, a molecular target drug, an immunostimulant (for example, a STING agonist (WO2020/050406, WO2014/099824, WO2014/179335, WO2014/189805, WO2014/189806, WO2015/074145, WO2015/185565, WO2016/096714, WO2016/012305, WO2016/145102, WO2017/027646, WO2017/027645, WO2017/075477, WO2017/093933, WO2017/100305, WO2017/123669, WO2017/161349, WO2017/175147, WO2017/175156, WO2018/009466, WO2018/045204, WO2018/060323, WO2018/067423, WO2018/065360, WO2014/093936, WO2018/009648, WO2018/100558), a TLR agonist, an A2AR antagonist, an IDO inhibitor, an antagonist to any one of CTLA-4, LAG-3 and PD-1 pathways, a checkpoint inhibitor, a vascular endothelial growth factor (VEGF) receptor inhibitor, a smoothen inhibitor, an alkylating agent, an antimetabolite, retinoid and an anticancer vaccine, an adjuvant, a lipid, a liposome, a toxin, an antibacterial agent, an antiviral agent, a diagnostic agent, a protein, a peptide, an amino acid, a nucleic acid, an antigen, a vitamin, a hormone)) to obtain an antibody (for example, an antibody-drug conjugate) further modified and having a desired activity. Examples of a chemotherapeutic agent or a toxin include camptothecin (for example, WO2014/057687), pyrrolobenzodiazepine (for example, WO2013/173496, WO2014/130879, WO2017/004330, WO2017/004025, WO2017/020972, WO2016/036804, WO2015/095124, WO2015/052322, WO2015/052534, WO2016/011519, WO2015/052321, WO2015/031693, WO2011/130613, WO2019/065964), doxorubicin, auristatin, taxane or a derivative thereof.

Examples of the donor molecule having an azide group ($N_3$-) as mentioned above include drug linkers described in WO2020/050406 and WO2019/065964. Examples of these include

N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spyro[cyclopropane-1,2'-pyrrolo[2,1-c][1,4]benzodiazepin]-10'(5'H)-yl]carbonyl}oxy)methyl]phenyl}-L-alaninamide,

N-[4-(11,12-didehydrodibenzo[b,f]azocin-5(6H)-yl)-4-oxobutanoyl]glycylglycyl-L-valyl-N-[4-({[(11'S,11'as)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spyro[cyclopropane-1,2'-pyrrolo[2,1-c][1,4]benzodiazepine]-10' (5'H)-carbonyl]oxy}methyl) phenyl]-L-alaninamide,

N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5', 11a'-dihydro-1'H-spyro[cyclopropane-1,2'-pyrrolo[2,1-c][1,4]benzodiazepin]-8'-yl]oxy}pentyl)oxy]-5'-oxo-11',11a'-dihydro-1'H-spyro[cyclopropane-1,2'-pyrrolo[2,1-c][1,4]benzodiazepin]-10'(5'H)-yl]carbonyl}oxy)methyl]phenyl}-L-alaninamide,
N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5', 10', 11',11a'-tetrahydro-1'H-spyro[cyclopropane-1,2'-pyrrolo[2,1-c][1,4]benzodiazepin]-8'-yl]oxy}pentyl)oxy]-5'-oxo-11',11a'-dihydro-1'H-spyro[cyclopropane-1,2'-pyrrolo[2,1-c][1,4]benzodiazepin]-10'(5'H)-yl]carbonyl}oxy)methyl]phenyl}-L-alaninamide, and
(bis(N,N-diethylethaneaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2$\lambda^5$, 10$\lambda^5$-flo[3,2-1][1,3,6,9,11,2,10]pentaoxadiphosphacyclotetradecin-7-yl]-6-oxo-6,9-dihydro-1H-purin-1-yl}ethoxy)methyl]glycinamide.

## Examples

Now, the present invention will be more specifically described by way of Examples. Examples show embodiments of the present invention and the present invention is not limited to these.

The protein concentrations described in the specification were measured by an ultralow volume spectrophotometer, NanoDrop1000 (manufactured by Thermo Fisher Scientific) or NanoDrop2000 (manufactured by Thermo Fisher Scientific).

A compound [$N_3$-PEG(3)]-MSG1(9)-Ox used in Examples is shown in Figure 1. A compound SGP is shown in Figure 2. A monoclonal antibody mAb1 is commercially available trastuzumab (purchased from Chugai Pharmaceutical Co. Ltd.). (Fucα1,6)GlcNAc-mAb1 refers to a hydrolysate of a sugar chain of trastuzumab. A monoclonal antibody, mAb2 refers to an antibody prepared in accordance with the method described in Example 136 of WO2019065964. The amino acid sequences of the light chain and heavy chain of mAb2 are represented by SEQ ID NO: 12 and SEQ ID NO: 13, respectively. (Fucα1,6)GlcNAc-mAb2 refers to a hydrolysate of a sugar chain, (Fucα1,6)GlcNAc-anti-CLDN6 antibody (H1L1) prepared in accordance with the method described in Example 61-step 1 of WO2019/065964.

The degrees of progress of sugar chain hydrolysis and transglycosylation reactions were checked by gel electrophoresis of a protein (Patent Literature 4, Non-Patent Literature 8). In the fully automated electrophoresis system for a protein, LabChip GX II (manufactured by PerkinElmer) was used as an apparatus; and Protein Express LabChip and Protein Express Reagent Kit (manufactured by PerkinElmer) were used as the reagents.

### <Example 1> Acquisition of *S. iniae*-derived endo-β-N-acetylglucosaminidase

A gene sequence of endo-β-N-acetylglucosaminidase was obtained from *S. iniae* SIO1002 strain by the following method.

First, genomic DNA was obtained from the bacterial cells of *S. iniae* SIO 1 002 strain inactivated with formalin (stain found in Japan, purchased from Kyoritsu Pharmaceutical Co., Ltd.). A 0.1% (w/v) formalin solution (1 mL) was centrifuged (6,000 rpm, 10 min, 4°C) and the precipitation was washed with sterile water (1 mL). Centrifugation was repeated in the same conditions and the precipitation was suspended in 200 μL of InstaGene DNA purification matrix (manufactured by Bio-Rad). The suspension was heated at 56°C for 30 minutes, followed at 99°C for 8 minutes and centrifuged (12,000 rpm, 10 min, 4°C). The supernatant obtained was used as a DNA extract.

A gene encoding endo-β-N-acetylglucosaminidase was amplified by using DNA in the extract as a template, primer 1 (SEQ ID NO: 14) and primer 2 (SEQ ID NO: 15), and PrimeSTAR Max DNA Polymerase (manufactured by Takara Bio Inc.) and the sequence of the gene was analyzed. The gene including a stop codon consists of 2787 nucleotides (SEQ ID NO: 1) and encodes a protein consisting of 928 amino acid residues (SEQ ID NO: 2) and having a molecular weight of 104,644. This protein was designated Endo-Si.

### <Example 2> Expression of Endo-Si by use of *Escherichia coli*

A nucleic acid sequence (SEQ ID NO: 16) optimized for expression in a heterologous cell such as *Escherichia coli.* was designed by adding a 6 × His tag to the C terminal of the endo-β-N-acetylglucosaminidase gene obtained in Example 1 and artificially synthesized by Eurofins Genomics. The artificially synthesized gene was cloned to pET24b (+) vector and transduced into *E. coli* BL21 (DE3). A transformed bacteria solution was inoculated into 2 mL of LB medium (1% (w/v) Tryptone, 0.5% (w/v) Yeast extract, 0.5% (w/v) NaCl, 50 μg/mL kanamycin) in a 12-mL conical tube, and incubated at 37°C overnight with shaking (600 rpm, O/N). This pre-culture solution (1.2 mL) was seeded in 100 mL of TB medium (1.2% (w/v) tryptone, 2.4% (w/v) yeast extract, 0.94% (w/v) $K_2HPO_4$, 0.22% (w/v) $KH_2PO_4$, 50 μg/mL

kanamycin, 0.01% (w/v) antifoam 204, 2 mM $MgSO_4$) contained in a 500-mL baffled flask and shaking culture was started at 37°C (210 rpm). After culture was carried out at 37°C for 1.5 hours, the temperature of the incubator was reduced to 16°C and culture was continued for one hour. After confirmation that the temperature of the culture solution reached 16°C, IPTG was added such that a final concentration became 0.2 mM and culture was further continued for 24 hours. After completion of the culture, bacterial cells were collected by centrifugation.

The bacterial cells collected were suspended in 5 mL of a binding buffer (50 mM HEPES (pH 8.0), 0.5 M NaCl, 20 mM imidazole, 5% (w/v) glycerol), homogenized by ultrasonic wave and centrifuged. The supernatant was purified by Ni Sepharose 6 Fast Flow (manufactured by GE Healthcare). The yield ($A_{280}$, absorption coefficient conversion) was 8.43 mg/100 mL broth.

<Example 3> Hydrolysis activity of Endo-Si on antibody sugar chain

The enzyme obtained in Example 2 was measured for hydrolysis activity by the following method. At this time, EndoS was used as a comparative subject.

In sterile water (5 mL), mAb 1 (60 mg) was dissolved. The solution was replaced with 50 mM Tris-HCl buffer (pH 7.5) while concentrating the solution by Vivaspin 20 (30,000 MWCO, PES, manufactured by Sartorius).

A reaction solution containing the mAb 1 (0.1 mg) and 10 ng of the enzyme (total volume 50 μL) was prepared and incubated at 37°C. Each of the reaction solutions at 0.5, 1, and 2 hours after initiation of the reaction was sampled and analyzed for protein by a fully automated electrophoresis system as mentioned above. In the chromatogram obtained, an unreacted product and a hydrolysate are confirmed as discrete peaks. From the peak-area ratio between the unreacted product and the hydrolysate, the hydrolysis rate of a sugar chain was calculated in accordance with the following expression.

$$\text{Hydrolysis rate of sugar chain (\%)} = [[\text{peak area of (Fuc}\alpha 1,6)\text{GlcNAc-mAb1-derived H-chain}]/\{[\text{peak area of mAb1-derived H-chain}]+[\text{peak area of (Fuc}\alpha 1,6)\text{GlcNAc-mAb1-derived H-chain}]\}] \times 100$$

A change with time in the reaction yield is shown in Figure 3. The hydrolysis rates of a sugar chain with Endo-Si and EndoS 0.5 hours after initiation of the reaction were 57.4% and 41.8%, respectively. Endo-Si exhibited a stronger hydrolysis activity than EndoS.

<Example 4> Study on reaction conditions for Endo-Si

The reaction temperature and pH for Endo-Si were investigated.

(4-1) Evaluation of reaction temperature for Endo-Si

The hydrolysis rates of a sugar chain with Endo-Si and EndoS at individual temperatures were measured as follows. A 50 mM Tris-HCl buffer (pH 7.5) solution (50 μL) containing 0.1 mg of mAb1 and 10 ng of Endo-Si WT or EndoS WT was prepared and incubated at each temperature of 27, 29, 31, 35, 37, 40, 43, 46, 48, and 50°C. Each of the reaction solutions at a half hour after initiation of a reaction was sampled and the hydrolysis rate of a sugar chain was calculated in accordance with the aforementioned method.

The results are shown in Figure 18. Endo-Si shows satisfactory hydrolysis activity at an optimum reaction temperature for hydrolysis (temperature at which an enzyme satisfactorily acts) within the range of 25°C to 45°C, more preferably 30°C to 42°C, particularly around 37°C (35°C to 39°C). It was confirmed that Endo-Si has higher hydrolysis activity than EndoS at individual temperatures.

(4-2) Evaluation of reaction pH of Endo-Si

The hydrolysis rates of a sugar chain with Endo-Si and EndoS at individual pH values were measured as follows. A 50 mM citric acid-sodium phosphate buffer (pH 5.0 or 5.5) solution, sodium phosphate buffer (pH 6.0, 6.5 or 7.0) solution or Tris-HCl buffer (pH 7.5, 8.0, 8.5 or 9.0) solution (50 μL for each) containing 0.1 mg of mAb1, and 10 ng of Endo-Si WT or EndoS WT, was prepared and incubated at 37°C. Each of the reaction solutions at a half hour after initiation of reaction was sampled and the hydrolysis rate of a sugar chain was calculated in accordance with the aforementioned method.

The results are shown in Figure 19. Endo-Si shows satisfactory hydrolysis activity at an optimum pH for hydrolysis (pH at which an enzyme satisfactorily acts) within the range of pH 6.3 to 9.0, more preferably pH 6.7 to 8.8, and particularly around pH 7.5 (pH 7.2 to 8.0).
It was confirmed that the activity of Endo-Si at around pH 6.7 or more is higher than that of EndoS.

<Example 5> Substrate specificity of Endo-Si

(5-1) Evaluation on substrate specificity of Endo-Si in various antibodies

The hydrolysis activities of Endo-Si, EndoS and PNGaseF on various antibodies were measured as follows. A 50 mM Tris-HCl buffer (pH 7.5) solution (50 μL) containing each of various substrates (10 μg), and 1 μg Endo-Si WT was prepared and incubated at 37°C. As the substrate, human IgG1-4, IgA, and IgE (all manufactured by Sigma Aldrich) were used. As the controls, 1 μg of EndoS WT and 500 U PNGase F PRIME (manufactured by N-zyme Scientifics) were used. Two hours after initiation of the reaction, a sample was taken from the reaction solution and analyzed for protein by a fully automated electrophoresis system mentioned above.

The results are shown in Figure 20. When a sugar chain is hydrolyzed by addition of an enzyme, the band of a protein shifts to the lower molecular weight side compared to when the enzyme was not added. As the result of the experiment, Endo-Si WT exhibited hydrolysis activity on all of the 4 subclasses of IgG but no hydrolysis activity on IgA and IgE. It was confirmed that the same substrate specificity was exhibited as in EndoS WT used as a control.

(5-2) Evaluation on substrate specificity of Endo-Si in various sugar chains

The substrate specificity of Endo-Si in various sugar chains were measured as follows. A 50 mM Tris-HCl buffer (pH 7.5) solution (10 μL) containing 5 pmol of each of various sugar chains labeled with 2-AB (manufactured by Agilent Technologies) and 20 μg of Endo-Si WT, was prepared and incubated at 37°C for 24 hours, and thereafter treated at 95°C for 5 minutes to terminate the reaction. The reaction solution was subjected to HPLC analysis carried out under the following conditions.

[HPLC analysis conditions]

HPLC apparatus: 1200 Infinity LC (manufactured by Agilent Technologies)
Column: ACQUITY UPLC Glycan Amide 130Å, 1.7 μm, 2.1 × 150 mm (manufactured by Waters)
Column temperature: 40°C
Detector: Fluorescence detector RF-20Axs (manufactured by Shimadzu Corporation)
Mobile phase A: $H_2O$ + 0.1% HCOOH
Mobile phase B: Acetonitrile + 0.1% HCOOH
Gradient (mobile phase B%): 90% (0 minute), 40% (25 minutes)
Flow rate: 0.2 mL/min

Enzyme activity was calculated based on the peak area ratio between a substrate and a hydrolysate, GlcNAc-2AB. The relative activities on various sugar chains when the activity on G0 sugar chain is regarded as 100% are shown in Table 4. Endo-Si exhibited activity on both of a high mannose-type sugar chain and a complex-type biantennary sugar chain but the specificity to (activity on) the complex-type biantennary sugar chain was higher than that to the high mannose-type sugar chain. Of them, the activity on (specificity to) G0 sugar chain is the highest. Endo-Si exhibits activity on a sialyl sugar chain and a fucosylated sugar chain but does not exhibit activity on a complex-type triantennary sugar chain.

[Table 4] Hydrolysis activity of Endo-Si on various sugar chains

[Table 4]

| Substrate | Structure | Relative activity | Substrate | Structure | Relative activity |
|---|---|---|---|---|---|
| 2-AB Man3 | 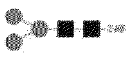 | 7.2% | 2-AB G0 (agalaclo biantennary) | 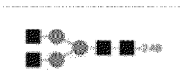 | 100% |
| 2-AB Man5 | 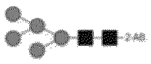 | 1.9% | 2-AB G2 (asialo biantennary) | 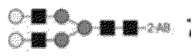 | 73.2% |
| 2-AB Man6 | 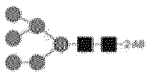 | 6.6% | 2-AB G2S2 (α2,6) (sialyl biantennary) | 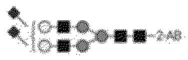 | 17.1% |
| 2-AB Man8 | 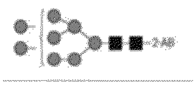 | 5.2% | 2-AB G2F (core-fucosyiated biantennary) | 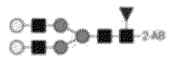 | 50.1 % |
| 2-AB Man9 | 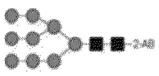 | 3.3% | 2-AB G3 (triantennary) | 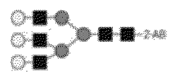 | Not detected |
| Relative activities were calculated based on the activity against G0 sugar chain regarded as 100%. ●: Mannose, ■: GlcNAc, ○: Galactose, ▼: fucose, ♦: Neu5Ac. | | | | | |

<Example 6> Modification of Endo-Si and measurement of transglycosylation activity

(6-1) Preparation of [$N_3$-PEG(3)]-MSG1(9)-Ox

[$N_3$-PEG(3)]-MSG1(9)-Ox used as a sugar chain donor in the following Examples was produced in accordance with the method described in Example 56 of WO2019/065964.

[Formula 6]

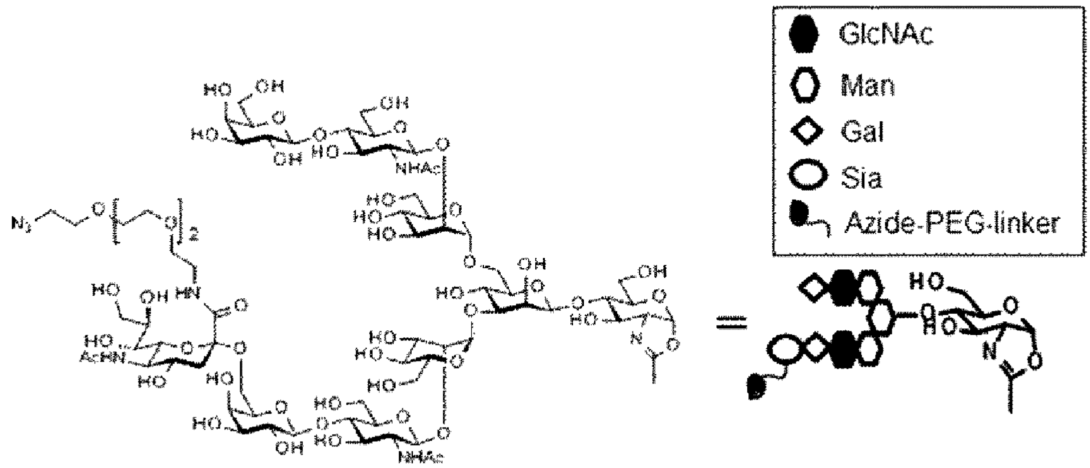


(6-2) Modification of Endo-Si and confirmation of transglycosylation activity

A mutation was introduced in order to obtain an Endo-Si mutant enzyme having high transglycosylation activity. Various mutant enzymes shown in Table 1 were designed based on the three-dimensional structure information (PDB ID: 4NUY) of EndoS and the transglycosylation activities of them on an antibody were measured.

Transglycosylation activity was evaluated as follows. A 50 mM Tris-HCl buffer (pH 7.5) solution (45 μL) containing (Fucα1,6)GlcNAc-mAb2 (0.5 mg), sugar-chain oxazoline [$N_3$-PEG(3)]-MSG1(9)-Ox (50.4 μg (8 eq.)) and an enzyme (1.25 μg) was prepared and incubated at 28°C. A sample was taken from each of the reaction solutions at 1, 2, 4, 6, and 24 hours after initiation of the reaction and analyzed for protein by a fully automated electrophoresis system

as mentioned above. In the chromatogram obtained, an unreacted product and transglycosylated molecule, mAb2-(MSG1-$N_3$)$_2$ were confirmed as discrete peaks. The transglycosylation rate was calculated based on the peak-area ratio between the unreacted product and the transglycosylated molecule in accordance with the following expression.

$$\text{Transglycosylation rate (\%)} = [[\text{peak area of mAb2-(MSG1-N}_3)_2\text{-derived H chain}]/\{[\text{peak area of (Fuc}\alpha 1,6)\text{GlcNAc-mAb2-derived H chain}] + [\text{peak area of mAb2-(MSG1-N}_3)_2\text{-derived H chain}]\}] \times 100$$

Similarly, the transglycosylation rates of individual Endo-Si mutant enzymes at each time period for a reaction were calculated (Table 5).

[Table 5] Change with time in transglycosylation rate of Endo-Si WT and mutant enzymes when sugar chain oxazoline was used as sugar chain donor

[Table 5]

| | Transglycosylation rate | | | | |
|---|---|---|---|---|---|
| | 1 h | 2 h | 4h | 6 h | 24 h |
| Endo-Si WT | 0.0 | 0.0 | 0.0 | 0.0 | 0.0 |
| D241Q | 75.2 | 97.0 | 99.1 | 99.6 | 98.8 |
| D241Q/Q311L | 5.7 | 11.6 | 27.4 | 44.4 | 99.3 |
| D241Q/E360Q | 73.5 | 96.8 | 99.0 | 99.6 | 98.9 |
| D241M | 98.8 | 98.9 | 97.4 | 97.5 | 75.0 |
| T190Q | 97.2 | 91.8 | 81.8 | 76.7 | 32.6 |
| D241M/Q311L | 31.8 | 59.1 | 95.9 | 99.5 | 99.9 |
| D241 M/E360Q | 99.1 | 99.0 | 96.2 | 95.5 | 75.9 |
| T190Q/D241Q | 26.6 | 48.4 | 83.3 | 96.1 | 99.6 |
| T1900/D241M | 43.5 | 64.1 | 92.7 | 98.7 | 99.6 |

Endo-Si mutant enzymes excluding Endo-Si WT all exhibited high transglycosylation activity.

<Example 7> Measurement of transglycosylation activity when SGP was used as a sugar chain donor

Transglycosylation activity was evaluated as follows. A 50 mM (sodium) phosphate buffer (pH 7.5) solution (17 μL) containing (Fucα1,6)GlcNAc-mAb2 (0.5 mg), SGP (0.485 mg (50 eq.)), Endo-M N175Q (manufactured by Tokyo Kasei Kogyo Co., Ltd.)(2.5 mU) and each of various Endo-Si mutant enzymes (10 μg), was prepared and incubated at 23°C. A sample was taken from each of the reaction solutions at 2, 4, 6, 24, and 48 hours after initiation of the reaction and analyzed for protein by a fully automated electrophoresis system as mentioned above. In the chromatogram obtained, an unreacted product and a transglycosylated molecule, mAb2-(SG)$_2$ are confirmed as discrete peaks. The transglycosylation rates were obtained based on the peak area ratio between the unreacted product and the transglycosylated molecule in accordance with the following expression.

$$\text{Glycosylation rate (\%)} = [[\text{peak area of mAb2-(SG)}_2\text{-derived H chain}]/\{[\text{peak area of (Fuc}\alpha\text{1,6)GlcNAc-mAb2-derived H chain}] + [\text{peak area of mAb2-(SG)}_2\text{-derived H chain}]\}] \times 100$$

Similarly, transglycosylation rates of individual Endo-Si mutant enzymes at each time period of a reaction were calculated (Table 6).

[Table 6] Change with time in transglycosylation rate of Endo-Si WT and mutant enzymes when SGP was used as a sugar chain donor

[Table 6]

| | Transglycosylation rate | | | | |
|---|---|---|---|---|---|
| | 2 h | 4h | 6h | 24 h | 48 h |
| Endo-Si WT | 1.2 | 1.1 | 0.0 | 0.0 | 0.0 |
| D241Q | 15.9 | 35.6 | 52.3 | 98.1 | 98.5 |
| D241Q/Q311L | 2.6 | 5.6 | 8.2 | 48.6 | 76.3 |
| D241Q/E360Q | 8.9 | 20.0 | 30.3 | 94.4 | 98.5 |
| D241M | 12.8 | 27.9 | 40.5 | 96.5 | 96.8 |
| T190Q | 13.3 | 27.8 | 43.2 | 93.8 | 93.3 |
| D241M/Q311L | 6.4 | 13.7 | 24.1 | 79.7 | 97.0 |
| D241M/E360Q | 11.9 | 27.7 | 42.7 | 98.0 | 98.7 |
| T190Q/D241Q | 4.9 | 10.6 | 21.4 | 71.5 | 92.5 |
| T190Q/D241M | 3.1 | 6.4 | 13.8 | 50.7 | 77.5 |

Endo-Si mutant enzymes excluding Endo-Si WT all exhibited high transglycosylation activity.

<Example 8> Study on the one-pot method by combination with Endo-Rp

Using Endo-Rp, which is known to transfer a sugar chain from a sugar chain donor, SGP, to a GlcNAc derivative, as a representative of enzyme A, the property of enzyme A that can be used in combination with Endo-Si was investigated.

The transglycosylation activity on an antibody by the one-pot method was evaluated as follows. A 50 mM (sodium) phosphorate buffer (pH 7.5) solution (17 μL) containing (Fucα1,6)GlcNAc-mAb2 (0.5 mg), SGP (0.485 mg (50 eq.)), Endo-MN175Q (manufactured by Tokyo Kasei Kogyo Co., Ltd.) (2.5 mU) or each of various Endo-Rp mutant enzymes (8 μg) and Endo-Si D241Q (5 μg) was prepared and incubated at 28°C. A sample was taken from each of the reaction solutions at 2, 4, 6, 24, and 48 hours after initiation of the reaction and analyzed for protein by a fully automated electrophoresis system as mentioned above. In the chromatogram obtained, the transglycosylation rate was calculated in accordance with the formula for computation described in Example 5.

The results are shown in Table 7. An Endo-Rp mutant enzyme having low activity to transfer a sugar chain from SGP to a GlcNAc derivative, for example, N172F, N172K, N172L, N172R, N172W, N172Y mutant enzymes having no transglycosylation activity as described in Patent Literature 3, exhibited low transglycosylation efficiency (activity) also in the one-pot method.

From the above, the transglycosylation activity of enzyme A, itself correlates to the sugar chain raw-material activation ability. Thus, it is considered that appropriate enzyme A having a sugar chain raw-material activation ability can be identified based on the transglycosylation activity from SGP to a GlcNAc derivative as an index. Generally, Endo-M, Endo-Om and Endo-CC are considered to have the same reactivity as Endo-Rp. Thus, enzyme A effective for the one-pot method can be identified by using Endo-M, Endo-Om or Endo-CC in place of Endo-Rp. Further, this method can be applied to Endo-S and Endo-S2 having a transglycosylation activity on an antibody similarly to Endo-Si.

As described above, the application range of the identification method mentioned above is not limited to a combination of Endo-Si and Endo-Rp and available to any combination of enzymes as long as individual enzymes have the same properties as those as mentioned above.

[Table 7] Change with time in transglycosylation activity of various mutant enzymes on an antibody using a one-pot method

[Table 7]

| Mutant | Change in transglycosylation rate (%) with time | | | | |
|---|---|---|---|---|---|
| | 2 h | 4 h | 6 h | 24 h | 48 h |
| N172Q | 17.3 | 32.5 | 44.0 | 94.8 | 97.4 |
| N172H | 9.1 | 14.9 | 20.3 | 83.7 | 99.3 |
| N172A | 26.7 | 50.0 | 66.2 | 95.9 | 97.8 |
| N172C | 50.8 | 79.6 | 91.8 | 95.2 | 87.8 |
| N172D | 7.0 | 9.1 | 9.4 | 8.2 | 6.2 |
| N172E | 7.7 | 15.5 | 23.0 | 83.8 | 94.8 |
| N172F | 0.0 | 0.0 | 0.0 | 0.0 | 0.0 |
| N172G | 17.8 | 32.0 | 44.2 | 92.8 | 96.8 |
| N172I | 3.9 | 5.7 | 7.6 | 29.2 | 71.2 |
| N172K | 0.0 | 0.0 | 0.0 | 0.5 | 0.0 |
| N172L | 0.0 | 0.0 | 0.0 | 2.1 | 3.1 |
| N172M | 4.2 | 8.1 | 11.2 | 49.3 | 82.7 |
| N172P | 4.0 | 8.2 | 11.8 | 55.3 | 90.2 |
| N172R | 0.0 | 0.0 | 0.0 | 0.0 | 0.0 |
| N172S | 36.5 | 62.1 | 78.4 | 93.2 | 85.8 |
| N172T | 44.4 | 72.7 | 87.8 | 96.5 | 94.4 |
| N172V | 28.3 | 55.0 | 76.7 | 97.9 | 97.7 |
| N172W | 0.1 | 0.0 | 0.0 | 0.0 | 0.0 |
| N172Y | 0.0 | 0.0 | 0.0 | 0.0 | 0.0 |
| W278FIS216V | 4.4 | 7.7 | 8.2 | 29.7 | 61.2 |
| W278F/N246D | 1.5 | 5.4 | 6.4 | 18.2 | 35.1 |
| W278F/D276N | 0.0 | 5.1 | 8.7 | 23.6 | 57.5 |
| W278F/A310D | 5.4 | 7.4 | 9.1 | 22.1 | 36.3 |
| W278F/N172D/F307Y | 12.0 | 19.0 | 24.9 | 76.5 | 93.1 |
| W278F/N172D/F307H | 8.8 | 13.4 | 17.7 | 66.1 | 91.1 |
| W278F/N172D/A310D | 0.0 | 0.0 | 0.0 | 8.5 | 17.0 |
| Y214F/F307Y/L3D6I | 34.5 | 53.0 | 63.4 | 87.4 | 88.8 |

<Example 9> Study on one-pot method using chemically modified sugar chain derivative as a donor

As a sugar chain derivative donor except SGP (donor) which is a natural sugar chain, a derivative, in which amino acids of a non-reducing end side and a reducing end side are modified with azide, was prepared and a transglycosylation reaction by a one-pot method was investigated.

(9-1) Preparation of ([$N_3$-PEG(3)]-MSG1-)Asn-PEG(3)-$N_3$

([$N_3$-PEG(3)]-MSG1-)Asn-PEG(3)-$N_3$ to be used as a sugar chain donor later in Examples was produced by the method described in Example 154, step 3 of WO2019065964.

[Formula 7]

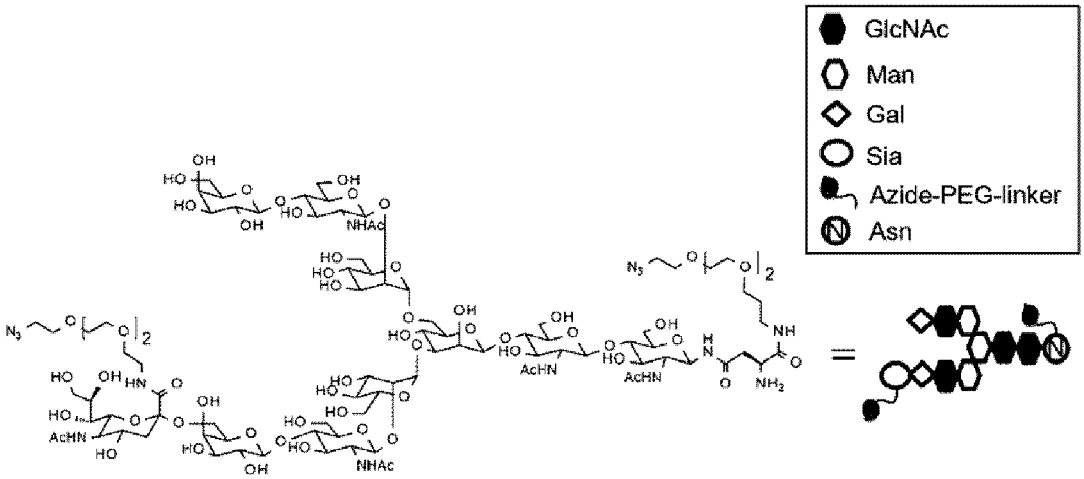


(9-2) Preparation of ([$N_3$-PEG(3)]$_2$-SG-)Asn-PEG(3)-$N_3$

([$N_3$-PEG(3)]$_2$-SG-)Asn-PEG(3)-$N_3$ to be used as a sugar chain donor later in Examples was produced by the method described in Example 1-12, step 1-12A of WO2018003983.

[Formula 8]

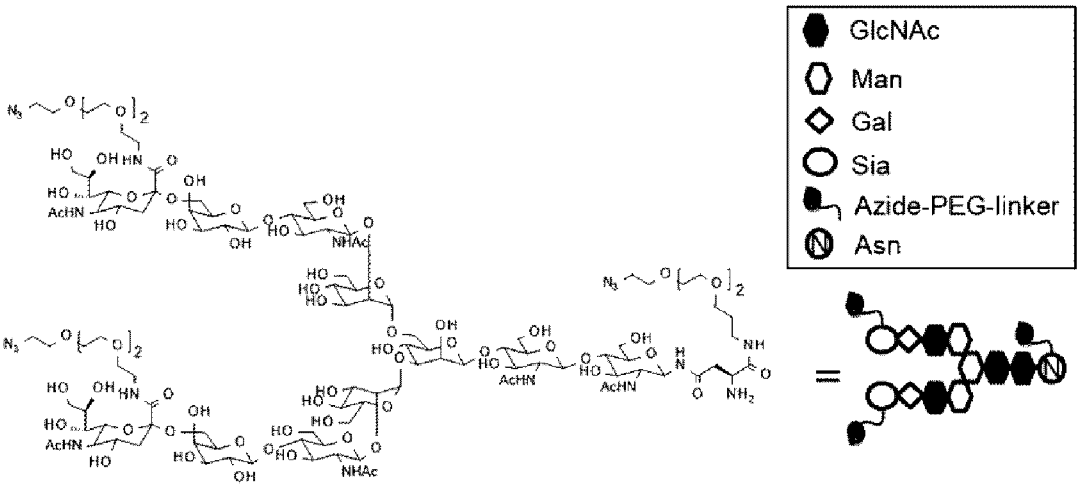


(9-3) Evaluation of transglycosylation activity

Transglycosylation activity was evaluated as follows. A 50 mM (sodium) phosphate buffer (pH 7.5) solution (17 μL) containing (Fucα1,6)GlcNAc-mAb2 (0.5 mg), SGP (0.485 mg (50 eq.)) or ([$N_3$-PEG(3)]-MSG1-)Asn-PEG(3)-$N_3$ (0.415 mg (50 eq.)) or ([$N_3$-PEG(3)]$_2$-SG-)Asn-PEG(3)-$N_3$ (0.495 mg (50 eq.)), Endo-Rp N172H (8 μg), and Endo-Si D241M/Q311L (10 μg), was prepared and incubated at 28°C. A sample was taken from the reaction solution at 2 hours, 4 hours, 6 hours, 24 hours, and 48 hours after initiation of the reaction and analyzed for protein by a fully automated electrophoresis system as mentioned above. When SGP was used as a donor, mAb2-(SG)$_2$ (Example 7) generated as

a transglycosylated molecule. When ([$N_3$-PEG(3)]-MSG1-)Asn-PEG(3)-$N_3$ was used as a donor, mAb2-(MSG1-$N_3$)$_2$ (Figure 21) generated as a transglycosylated molecule. When ([$N_3$-PEG(3)]$_2$-SG-)Asn-PEG(3)-$N_3$ was used as a donor, mAb2-[SG-($N_3$)$_2$]$_2$ (Figure 22, wherein it is referred to as mAb2-(SG-$N_3$)$_2$) generated as a transglycosylated molecule. In the chromatogram obtained, an unreacted product and individual transglycosylated molecules were confirmed as discrete peaks. When SGP was used as a donor, the transglycosylation rate was calculated in accordance with the formula for computation described in Example 5. When ([$N_3$-PEG(3)]-MSG1-)Asn-PEG(3)-$N_3$ was used as a donor, the transglycosylation rate was calculated in accordance with the formula for computation described in Example 4-2. When ([$N_3$-PEG(3)]$_2$-SG-)Asn-PEG(3)-$N_3$ was used as a donor, the transglycosylation rate was calculated in accordance with the following formula for computation.

$$\text{Transglycosylation rate (\%)} = [[\text{peak area of mAb2-(SG-N}_3)_2\text{-derived H chain}]/\{[\text{peak area of (Fuc}\alpha 1,6)\text{GlcNAc-mAb2-derived H chain}] + [\text{peak area of mAb2-(SG-N}_3)_2\text{-derived H chain}]\}] \times 100$$

The results are shown in Table 8. It was confirmed that when not only a natural-type sugar chain structure but also sugar chain chemically modified was used as a donor, a one-pot transfer reaction proceeded.

[Table 8] Change with time in transglycosylation activity of various sugar chain donors on an antibody by one-pot method.

[Table 8]

| Sugar chain donor | Transglycosylation rate (%) | | | | |
|---|---|---|---|---|---|
| | 2 h | 4 h | 6 h | 24 h | 48 h |
| SGP | 3.8 | 5.0 | 11.2 | 53.5 | 89.7 |
| ([$N_3$-PEG(3)]-MSG1-)Asn-PEG(3)-$N_3$ | 6.6 | 13.6 | 19.9 | 83.5 | 99.0 |
| ([$N_3$-PEG(3)]$_2$-SG-)Asn-PEG(3)-$N_3$ | 5.0 | 12.5 | 16.2 | 62.3 | 94.0 |

Industrial Applicability

An antibody having homogeneous sugar chains obtained by the Endo-Si enzyme of the present invention or a sugar chain-containing molecule can be obtained efficiently or with a high purity and used as a medicine.

Sequence listing free text

SEQ ID NO: 1: Endo-Si nucleotide sequence
SEQ ID NO: 2: Endo-Si amino acid sequence
SEQ ID NO: 3: Endo-Si amino acid sequence D241Q
SEQ ID NO: 4: Endo-Si amino acid sequence D241Q/Q311L
SEQ ID NO: 5: Endo-Si amino acid sequence D241Q/E360Q
SEQ ID NO: 6: Endo-Si amino acid sequence D241M
SEQ ID NO: 7: Endo-Si amino acid sequence D241M/Q311L
SEQ ID NO: 8: Endo-Si amino acid sequence D241M/E360Q
SEQ ID NO: 9: Endo-Si amino acid sequence T190Q/D241Q
SEQ ID NO: 10: Endo-Si amino acid sequence T190Q
SEQ ID NO: 11: Endo-Si amino acid sequence T190Q/D241M
SEQ ID NO: 12: mAb2 light-chain amino acid sequence
SEQ ID NO: 13: mAb2 heavy-chain amino acid sequence
SEQ ID NO: 14: Primer 1
SEQ ID NO: 15: Primer 2

SEQ ID NO: 16: Endo-Si sequence for *E. coli*
SEQ ID NO: 17: Endo-Rp amino acid sequence N172Q
SEQ ID NO: 18: Endo-Rp amino acid sequence N172H
SEQ ID NO: 19: Endo-Rp amino acid sequence N172A
SEQ ID NO: 20: Endo-Rp amino acid sequence N172C
SEQ ID NO: 21: Endo-Rp amino acid sequence N172D
SEQ ID NO: 22: Endo-Rp amino acid sequence N172E
SEQ ID NO: 23: Endo-Rp amino acid sequence N172F
SEQ ID NO: 24: Endo-Rp amino acid sequence N172G
SEQ ID NO: 25: Endo-Rp amino acid sequence N1721
SEQ ID NO: 26: Endo-Rp amino acid sequence N172K
SEQ ID NO: 27: Endo-Rp amino acid sequence N172L
SEQ ID NO: 28: Endo-Rp amino acid sequence N172M
SEQ ID NO: 29: Endo-Rp amino acid sequence N172P
SEQ ID NO: 30: Endo-Rp amino acid sequence N172R
SEQ ID NO: 31: Endo-Rp amino acid sequence N172S
SEQ ID NO: 32: Endo-Rp amino acid sequence N172T
SEQ ID NO: 33: Endo-Rp amino acid sequence N172V
SEQ ID NO: 34: Endo-Rp amino acid sequence N172W
SEQ ID NO: 35: Endo-Rp amino acid sequence N172Y
SEQ ID NO: 36: Endo-Rp amino acid sequence W278F/S216V
SEQ ID NO: 37: Endo-Rp amino acid sequence W278F/N246D
SEQ ID NO: 38: Endo-Rp amino acid sequence W278F/D276N
SEQ ID NO: 39: Endo-Rp amino acid sequence W278F/A310D
SEQ ID NO: 40: Endo-Rp amino acid sequence W278F/N172D/F307Y
SEQ ID NO: 41: Endo-Rp amino acid sequence W278F/N172D/F307H
SEQ ID NO: 42: Endo-Rp amino acid sequence W278F/N172D/A310D
SEQ ID NO: 43: Endo-Rp amino acid sequence W278F/F307Y/L306I
SEQ ID NO: 44: Endo-M amino acid sequence
SEQ ID NO: 45: Endo-Om amino acid sequence
SEQ ID NO: 46: Endo-CC amino acid sequence

All publications, patents and patent applications are incorporated herein in their entireties by reference.

## Claims

1. A polypeptide having an amino acid sequence at amino acid positions 34 to 928 in SEQ ID NO: 2 or an amino acid sequence having the same amino acid sequence except containing one or more mutations at one or more amino acid positions selected from the group consisting of amino acids at position 241 (D241), position 190 (T190), position 311 (Q311) and position 360 (E360), said polypeptide exhibiting a sugar chain hydrolysis activity and/or transglycosylation activity.

2. The polypeptide according to claim 1, having one or more mutations at 1 to 3 amino acid positions selected from the group consisting of amino acids of D241, T190, Q311 and E360.

3. The polypeptide according to claim 1 or 2, having one or more mutations selected from the group consisting of the following (A) to (D):

   (A) a mutation of the amino acid at position 241 (D241) to glutamine (D241Q), methionine (D241M) or alanine (D241A) in the amino acid sequence of SEQ ID NO: 2;
   (B) a mutation of the amino acid at position 190 (T190) to glutamine (T190Q) in the amino acid sequence of SEQ ID NO: 2;
   (C) a mutation of the amino acid at position 311 (Q311) to leucine (Q311L) in the amino acid sequence of SEQ ID NO: 2; and
   (D) a mutation of the amino acid at position 360 (E360) to glutamine (E360Q), alanine (E360A), asparagine (E360N) or aspartic acid (E360D) in the amino acid sequence of SEQ ID NO: 2.

**4.** The polypeptide according to any one of claims 1 to 3, having one or more mutations selected from the group consisting of the following (A) to (D):

(A) D241Q or D241M;
(B) T190Q;
(C) Q311L; and
(D) E360Q.

**5.** The polypeptide according to any one of claims 1 to 4, comprising any one of the following amino acid sequences (A) to (C):

(A) an amino acid sequence selected from the group consisting of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11;
(B) an amino acid sequence having a homology or identity of at least 90% to each of the amino acid sequences defined in (A) excluding an amino acid at position 241, 190, 311 or 360; or
(C) an amino acid sequence having deletion, substitution and/or addition of one or several amino acids relative to the sequence defined in (A) excluding an amino acid at position 241, 190, 311 or 360.

**6.** The polypeptide according to any one of claims 1 to 5, exhibiting hydrolysis activity and/or transglycosylation activity on an N-linked sugar chain.

**7.** The polypeptide according to claim 6, wherein the N-linked sugar chain is an N-linked sugar chain in a glycoprotein.

**8.** The polypeptide according to claim 6 or 7, wherein the glycoprotein is an antibody or a molecule containing an Fc region of an antibody (an Fc region-containing molecule).

**9.** The polypeptide according to any one of claims 6 to 9, wherein the N-linked sugar chain is an N-linked sugar chain linked to Asn at position 297 of an antibody (an N297-linked sugar chain).

**10.** The polypeptide according to claim 9, wherein the N297-linked sugar chain is a complex-type sugar chain having a non-reducing end optionally modified chemically.

**11.** The polypeptide according to claim 9 or 10, wherein the N297-linked sugar chain is an N297-linked sugar chain optionally having fucose added to core GlcNAc.

**12.** A polynucleotide encoding the polypeptide according to any one of claims 1 to 11.

**13.** An expression vector comprising the polynucleotide according to claim 12.

**14.** A host cell transformed with the expression vector according to claim 13.

**15.** A production method for the polypeptide according to any one of claims 1 to 11, comprising a step of culturing the host cell according to claim 14 and a step of collecting a polypeptide of interest from a culture obtained in the culturing step.

**16.** A polypeptide obtained by the production method according to claim 15.

**17.** A production method for an antibody or its Fc region-containing molecule, comprising reacting an acceptor molecule, which is an antibody or its Fc region-containing molecule having, as an N297-linked sugar chain, core GlcNAc optionally having fucose added thereto, with a sugar-chain donor molecule containing GlcNAc having a reducing end activated, in the presence of the polypeptide according to any one of claims 1 to 11.

**18.** The production method according to claim 17, wherein the GlcNAc having a reducing end activated is oxazolylated GlcNAc.

**19.** The production method according to claim 17 or 18, wherein the sugar-chain donor molecule is a complex-type sugar chain having a non-reducing end optionally modified chemically.

**20.** The production method according to any one of claims 17 to 19, wherein the sugar-chain donor molecule is SG(10)-Ox, MSG1(9)-Ox, MSG2(9)-Ox or a mixture of MSG1(9)-Ox and MSG2(9)-Ox, having a non-reducing end optionally modified chemically.

**21.** The production method according to any one of claims 17 to 20, wherein the sugar-chain donor molecule is [$N_3$-PEG(3)]$_2$-SG(10)-Ox, [$N_3$-PEG(3)]-MSG1(9)-Ox, [$N_3$-PEG(3)]-MSG2(9)-Ox or a mixture of [$N_3$-PEG(3)]-MSG1(9)-Ox and [$N_3$-PEG(3)]-MSG2(9)-Ox.

**22.** The production method according to claim 21, further comprising a step of reacting the azide group ($N_3$-) with a molecule having an alkyne structure.

**23.** The production method according to claim 22, wherein the molecule having an alkyne structure is selected from a chemotherapeutic agent, a molecular target drug, an immunostimulant, a toxin, an antibacterial agent, an antiviral agent, a diagnostic agent, a protein, a peptide, an amino acid, a nucleic acid molecule, a nucleic acid, an antigen, a lipid, a liposome, a vitamin and a hormone.

**24.** The production method according to claim 23, wherein the chemotherapeutic agent is selected from camptothecin, pyrrolobenzodiazepine, doxorubicin, auristatin, taxane and a derivative thereof.

**25.** The production method according to claim 23, wherein the immunostimulant is selected from a STING agonist, a TLR agonist, an A2AR antagonist, an IDO inhibitor, an antagonist to any one of CTLA-4, LAG-3 and PD-1 pathways, a checkpoint inhibitor, a vascular endothelial growth factor (VEGF) receptor inhibitor, a smoothen inhibitor, an alkylating agent, an antimetabolite, retinoid, an anticancer vaccine and an adjuvant.

**26.** The production method according to any one of claims 23 to 25, wherein the molecule having an alkyne structure is selected from the group consisting of (A) to (E):

(A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;
(B) N-[4-(11,12-didehydrodibenzo[b,f]azocin-5(6H)-yl)-4-oxobutanoyl]glycylglycyl-L-valyl-N-[4-({[(11'S,11'as)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide;
(C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;
(D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide; and
(E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R, 12aR, 14R, 15R, 15aR, 16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2$\lambda^5$,10$\lambda^5$-furo[3,2-1][1,3,6,9,11,2,10]pentaoxadiphosphacyclotetradecyn-7-yl]-6-oxo-6,9-dihydro-1H-purin-1-yl} ethoxy)methyl]glycinamide.

**27.** The production method according to any one of claims 17 to 26, wherein the acceptor molecule is an antibody or Fc region-containing molecule having an N297-linked sugar chain consisting of core GlcNAc optionally having fucose added.

**28.** A production method for an antibody or an Fc region-containing molecule, comprising reacting an acceptor molecule, which is an antibody or its Fc region-containing molecule having, as an N297-linked sugar chain, core GlcNAc optionally having fucose added, with a sugar-chain donor molecule containing GlcNAc having a reducing end not

activated, in the presence of the polypeptide according to any one of claims 1 to 11 and endo-β-N-acetylglucosaminidase (Enzyme A) that prefers a complex-type sugar chain of a sugar chain donor molecule having a reducing end not activated as a substrate but does not prefer an N297-linked sugar chain as a substrate.

**29.** The production method according to claim 28, comprising reacting the polypeptide according to any one of claims 1 to 11, Enzyme A, the acceptor molecule and the sugar-chain donor molecule in a single reaction solution.

**30.** The production method according to claim 28 or 29, wherein the sugar-chain donor molecule is a complex-type sugar chain having a non-reducing end optionally modified chemically.

**31.** The production method according to any one of claims 28 to 30, wherein the sugar-chain donor molecule is SGP, (SG-)Asn, (MSG1-)Asn, (MSG2-)Asn or a mixture of (MSG1-)Asn and (MSG2-)Asn, having a non-reducing end optionally modified chemically.

**32.** The production method according to any one of claims 28 to 31, wherein the sugar-chain donor molecule is ([$N_3$-PEG(3)]$_2$-SG-)Asn-PEG(3)-$N_3$, ([$N_3$-PEG(3)]-MSG1-)Asn-PEG(3)-$N_3$, ([$N_3$-PEG(3)]-MSG2-)Asn-PEG(3)-$N_3$ or a mixture of ([$N_3$-PEG(3)]-MSG1-)Asn-PEG(3)-$N_3$ and ([$N_3$-PEG(3)]-MSG2-)Asn-PEG(3)-$N_3$.

**33.** The production method according to claim 32, further comprising a step of reacting the azide group ($N_3$-) with a molecule having an alkyne structure.

**34.** The production method according to claim 33, wherein the molecule having an alkyne structure is selected from a chemotherapeutic agent, a molecular target drug, an immunostimulant, a toxin, an antibacterial agent, an antiviral agent, a diagnostic agent, a protein, a peptide, an amino acid, a nucleic acid, an antigen, a lipid, a liposome, a vitamin and a hormone.

**35.** The production method according to claim 34, wherein the chemotherapeutic agent is selected from camptothecin, pyrrolobenzodiazepine, doxorubicin, auristatin, taxane and a derivative thereof.

**36.** The production method according to claim 35, wherein the immunostimulant is selected from a STING agonist, a TLR agonist, an A2AR antagonist, an IDO inhibitor, an antagonist to any one of CTLA-4, LAG-3 and PD-1 pathways, a checkpoint inhibitor, a vascular endothelial growth factor (VEGF) receptor inhibitor, a smoothen inhibitor, an alkylating agent, an antimetabolite, retinoid, an anticancer vaccine and an adjuvant.

**37.** The production method according to any one of claim 34 to 36, wherein the molecule having an alkyne structure is selected from the group consisting of (A) to (E):

(A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spirofcyclopropane-1,2'-pyrrolo[2,1-c][1,4]benzodiazepin]-10'(5'H)-yl]carbonyl}oxy)methyl]phenyl}-L-alaninamide;
(B) N-[4-(11,12-didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide;
(C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide;
(D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide; and
(E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R,12aR,14R,15R,15aR, 16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-

2λ5,10λ5-furo[3,2-1][1,3,6,9,11,2,10]pentaoxadiphosphacyclotetradecyn-7-yl]-6-oxo-6,9-dihydro-1H-purin-1-yl} ethoxy)methyl]glycinamide.

**38.** The production method according to any one of claims 28 to 37, wherein the acceptor molecule is an antibody or Fc region-containing molecule having an N297-linked sugar chain consisting of core GlcNAc optionally having fucose added.

**39.** The production method according to any one of claims 28 to 38, wherein Enzyme A is an enzyme having transglycosylation activity from SGP to an acceptor having GlcNAc.

**40.** The production method according to any one of claims 28 to 39, wherein Enzyme A is any one of Endo-M, Endo-Rp, Endo-Om, Endo-CC and a mutant enzyme thereof having a reduced hydrolysis activity.

**41.** The production method according to claim 40, wherein the mutant enzyme having a reduced hydrolysis activity is selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-CC N180H and Endo-Om N194Q.

**42.** An antibody or Fc region-containing molecule obtained by the production method according to any one of claims 17 to 41.

**43.** A production method for an antibody or Fc region-containing molecule having only core GlcNAc optionally having fucose added, comprising reacting an antibody or Fc region-containing molecule with a polypeptide having an amino acid sequence at amino acid positions 34 to 928 in SEQ ID NO: 2.

**44.** An antibody or Fc region-containing molecule having only core GlcNAc obtained by the production method according to claim 43.

[Fig. 1]

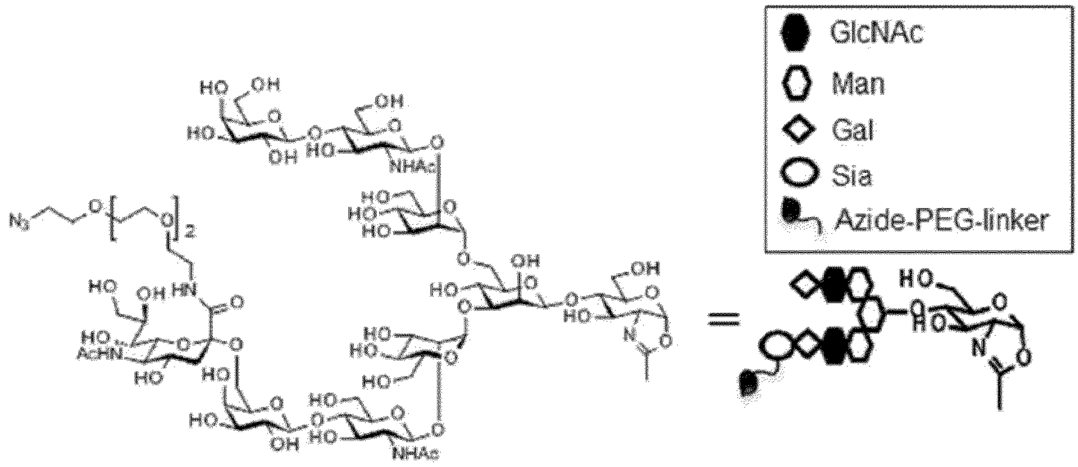


[Fig. 2]

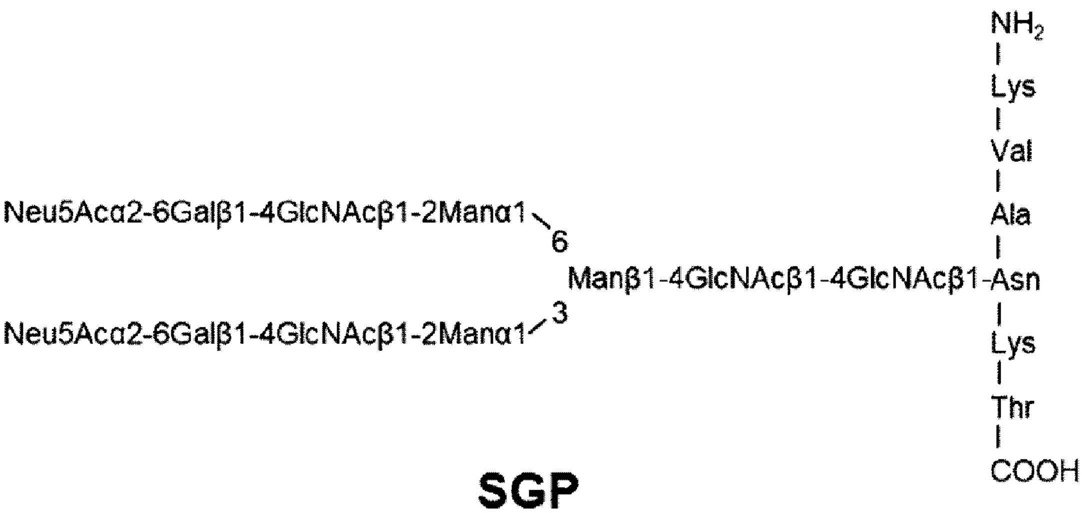


**SGP**

[Fig. 3]

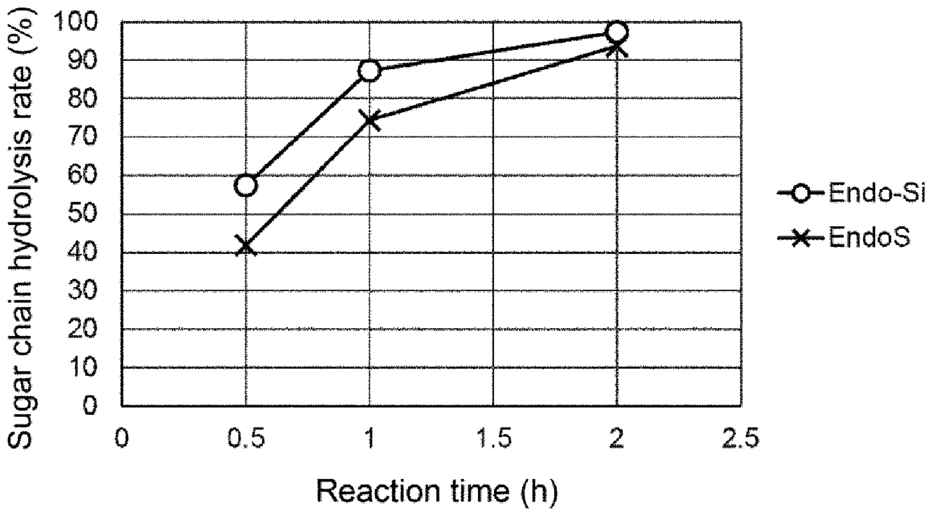
Sugar chain hydrolysis rate (%)
100
90
80
70
60
50
40
30
20
10
0
0
0.5
1
1.5
2
2.5
Reaction time (h)
Endo-Si
EndoS

[Fig. 4]

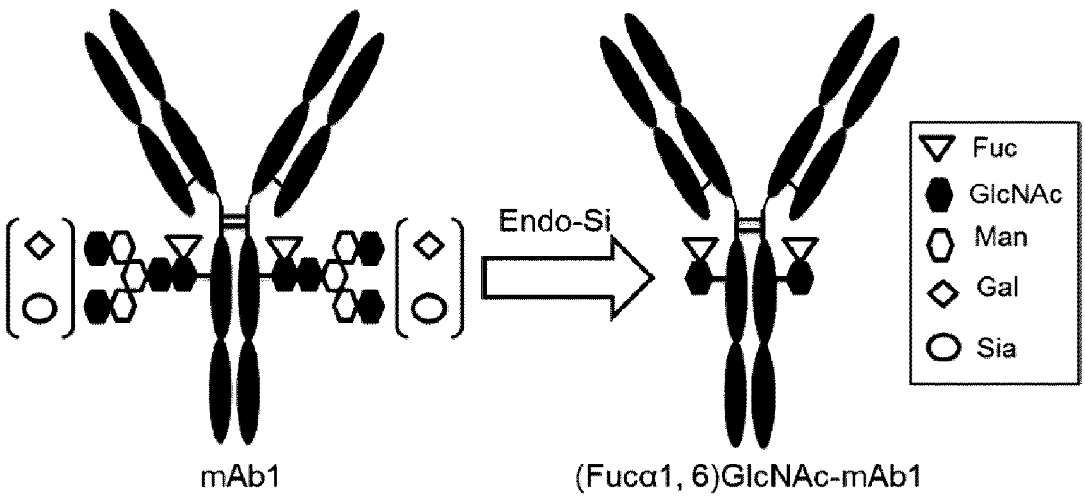
Endo-Si
mAb1
(Fucα1, 6)GlcNAc-mAb1
Fuc
GlcNAc
Man
Gal
Sia

[Fig. 5]

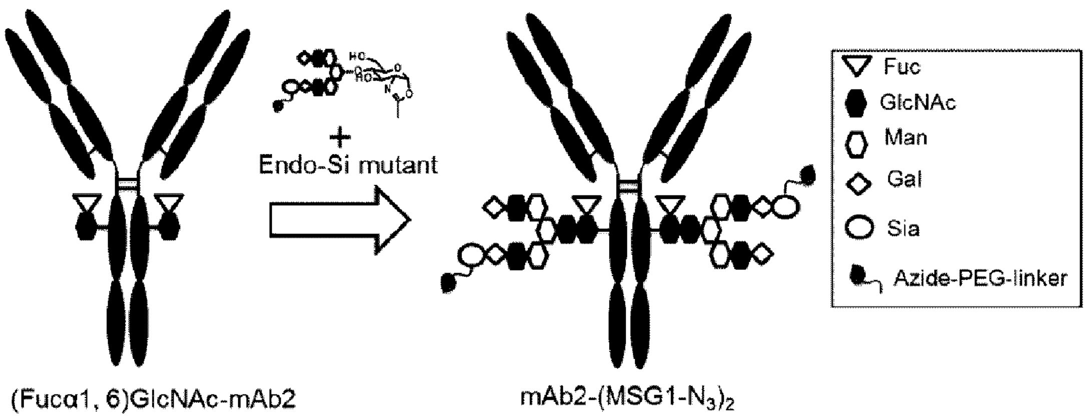
+
Endo-Si mutant
Fuc
GlcNAc
Man
Gal
Sia
Azide-PEG-linker
(Fucα1, 6)GlcNAc-mAb2
mAb2-(MSG1-N3)2

[Fig. 6]

SEQ ID NO: 1 Endo-Si nucleotide sequence

ATGAACAAACGTTTATTGGTTAAACGCACTTTCGGTTGTGTCTGCGCAGCAGCTATTTTAGG
TGTTGCCCCCCTCAGCCATCCAACAATCGTCGAGGCAAGAGAAGAATTGAAGATGCCAAACG
GCCTTGAACAATCAATTGCTGACGTCGAAGCTAAAATTGATGCCTTAACATATCTTTCAAAG
AATAGTAAAGATGAATTTAAGCATTCCATGTATGAAATCCCGTCAAATCGTGAACACAAACC
AGTATCACCCAAACAGGCCTTGCAAAACGCTAAAAAAGCTGATGCTCAAGCAGAACGCCTTG
CCAAAATGACCATTCCTAAAAAGGAAGAACTAAAAGCACTCGAAGGACCACTTTACGGTGGC
TATTTCCGTACCTGGCAAGATAAAACTTCTGATCCTACTGAAACTAATAAGGTCAACTCCTT
TGGGGAGTTGCCTAAAGAAGTTGACCTAGCATTTGTTTTTCACGACTACACTAAAGACTATA
GCCTTTTCTGGGAGGAATTAGCGACAAAACAAGTTCCGAAACTCAACAAGCAACGAACACGT
GTGATTCGTACCATTCCATGGCGTTTCTTAAGTGGTGCTGACCATAGTGATATCTCTGCTGA
TAAGGAGAAATTCCCTAACACTGAAGCTGGAAACAAAGCACTGGCTAAAGCTATCGTTGATG
AATATGTTTACAAATACAACCTTGATGGCTTAGACATCGATATCGAACGTGATAGTGTTCCT
AAAGTTAATGACAAAGAAGATCCCGAAGCACTGGCTCGCACCGTTGAAGTCTTTAAAGAAAT
CGGCAAATTAATTGGTGCAAATGGCGCTGACAAGAGCCGCCTCTTAATCATGGACACAACCT
ATACTGCTGAAGAAAACCCATTGATTAAAGAAACAGCACAATACCTAAACTTACTCTTGGTT
CAAGTCTATGGCTTCTCTGGAGAAAATGGAAATTATTTACATCACAAGAACATATTAGACGA
AACAAGTAGCATGGAAGGCAGATGGCAAGGCTATAGCAAATACATTCGTCCAGAACAATACA
TGGTCGGCTTTTCATTCTATGAAGAAAAGGATTTCAATAATCGTTGGAAAGATATTAATGAA
GAAGATCCTTCCGATCCACATATTGGTGAGAAAATCCAAGGAACGCGTGCTGAACGGTATGC
TAAGTGGCAACCTAAAACAGGTGGGCTAAAAGGTGGTCTCTTCTCTTATGCCATTGACCGCG
ATGGGGTTGCACAACCAAAACAAAAAACTGAGCACCCAGAACTAGACAAGATCGTTAAATCT
GAATACAAAGTATCTAAAGCTTTGAAAAAACTCATCATGACAGATGACCAATACCAACCAAT
TGATCAATCTGATTTTCCTGATAAGGCACTCCGTGAAAGCATTATTAAACAGGTCGGGACAA
GACGTGGCGACTTAGAACGTTTCAAGGGTACTCTAAGACTTGACAATCCTGAGATTAAAGAT
TTGACAGGTCTTAACAAACTTAAAAGAGTCGCTAAACTAGAACTCATCAACCTTCCAAAAAT
CACTAAAATTGATAAGGACGATCTCCCACAAAACCTTAAACCTTTAACTGACAATCAAAAAT
CTAACCTCGAAATAAAAGGCACTTACGATGATTCAAAACTATATAAGGATATTCCAGCCTTT
GATTTGGTCATTTCTGGTCTAAGTGGTCTTGAATCATTGGATATTTCAGGCCATCAGCGTGA
TACACTTAGTGGTATTGATGCTTCCACACTTCCTTCTTTAAAAGCAATCAATATTTCTGATA
ATCACTTTGATTTGGCACAAGGAACTGAAAATCGTCACATTCTTGATACTATCTTAGCTACA
CTTGCTAAAAATGGTGCCTCAACAGCTAGCTTTCATAAACAAAAACCAAAAGGCCTATACCC
TGAAAGCTATAGTACTGCCCCACTTCACCTCCAAGTTGGCCAAGGTAAAATCAATGTCATTG
ATGACCTTATCTTTGGAACTCGCACCAATCAAAATACCTTAATTAATACTGAAAATGACTTT
GAGGCCTATAAAGAGCAAACCATTCAGGGTAAACCTTTTATTGCCCCTGATTATCTCTATGA
CAACTTTAAAGTTAGTTATAAGGAATACTCTGCTTCAATCGTTGACTCAACACTTGCTGAAA
CAACTGATAAAACCATTGATACCGCTAAAGCTGAAACTTATCAGGTCACTGTCTCAAACAAG
GATGGAAAAACTGTTCACTCAGTTAAAGTTATCGTTGGTGACGAAAAACCCATGATGGTTAA
CTTAGCACAACATGCAAAAATCATTGCTACTGACAACATCACTCAAAGTGCAAAAGTTTTTC
ATGGGCAAAAAGACCAATTTCTTTTAAGTTGGAATAAAGACTCCTCTGTCATTTTTGAATTA
AAAACCCCTGGTACAGCCAAACACTGGCGCTTCTTTGATGATGGCAAGAATGACTCTGTAAC
CCTATCTGTCTTCAAAGGGGATGCATCTAACTTTGAAACTGAAAAAGACAAAGCCGAAAATT
GGGTTGAAATCACAAAAGACAGCCGCAAAAATGACGACAAAGTATTCAGTAGTCCATTAGAA
GTTGACAATGCCAAATATTTGAAAGTGACAATAAAAAAAGAAGCTAAGTATATCTACTTCAA
CGAACTTCAAATCCTTGGTTATCCAGGTGTAGTTGCTAAAAAAACTGCTGATGACCTCAGAC
CAACAGAGGCAGACAAGAGCGATGACAAATCCGACAAAAATGACACAGAAGCCAAGTAA

[Fig. 7]

SEQ ID NO: 2 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIDIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 8]

SEQ ID NO: 3 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIQIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 9]

SEQ ID NO: 4 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIQIERDSVP
KVNDKEDPEALARTVEVFKEIGKLIGANGADKSRLLIMDTTYTAEENPLIKETAQYLNLLLV
LVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 10]

SEQ ID NO: 5 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIQIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEQKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 11]

SEQ ID NO: 6 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIMIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 12]

SEQ ID NO: 7 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIMIERDSVP
KVNDKEDPEALARTVEVFKEIGKLIGANGADKSRLLIMDTTYTAEENPLIKETAQYLNLLLV
LVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 13]

SEQ ID NO: 8 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRTIPWRFLSGADHSDISADKEKFPNTEAGNKALAKAIVDEYVYKYNLDGLDIMIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEQKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 14]

SEQ ID NO: 9 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRQIPWRFLSGADHSDISADKEKFPNTEAGNKALAKAIVDEYVYKYNLDGLDIQIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 15]

SEQ ID NO: 10 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRQIPWRFLSGADHSDISADKEKFPNTEAGNKALAKAIVDEYVYKYNLDGLDIDIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 16]

SEQ ID NO: 11 Endo-Si amino acid sequence

MNKRLLVKRTFGCVCAAAILGVAPLSHPTIVEAREELKMPNGLEQSIADVEAKIDALTYLSK
NSKDEFKHSMYEIPSNREHKPVSPKQALQNAKKADAQAERLAKMTIPKKEELKALEGPLYGG
YFRTWQDKTSDPTETNKVNSFGELPKEVDLAFVFHDYTKDYSLFWEELATKQVPKLNKQGTR
VIRQIPWRFLSGADHSDISADKEKFPNTEAGNKALAKAIVDEYVYKYNLDGLDIMIERDSVP
KVNDKEDPEALARTVEVFKEIGKLIGANGADKSRLLIMDTTYTAEENPLIKETAQYLNLLLV
QVYGFSGENGNYLHHKNILDETSSMEGRWQGYSKYIRPEQYMVGFSFYEEKDFNNRWKDINE
EDPSDPHIGEKIQGTRAERYAKWQPKTGGLKGGLFSYAIDRDGVAQPKQKTEHPELDKIVKS
EYKVSKALKKLMMTDDQYQPIDQSDFPDKALRESIIKQVGTRRGDLERFKGTLRLDNPEIKD
LTGLNKLKRVAKLELINLPKITKIDKDDLPQNLKPLTDNQKSNLEIKGTYDDSKLYKDIPAF
DLVISGLSGLESLDISGHQRDTLSGIDASTLPSLKAINISDNHFDLAQGTENRHILDTILAT
LAKNGASTASFDKQKPKGLYPESYSTAPLHLQVGQGKINVIDDLIFGTRTNQNTLINTENDF
EAYKEQTIQGKPFIAPDYLYDNFKVSYKEYSASIVDSTLAETTDKTIDTAKAETYQVTVSNK
DGKTVHSVKVIVGDEKPMMVNLAQDAKIIGTDNMTQSAKVFDGQKDQFLLSWNKDSSVIFEL
KTPGTAKHWRFFDDGKNDSVTLSVFKGDASNFETEKDKAENWVEITKDSRKNDDKVFSSPLE
VDNAKYLKVTIKKEAKYIYFNELQILGYPGVVAKKTADDLRPTEADKSDDKSDKNDTEAK

[Fig. 17]

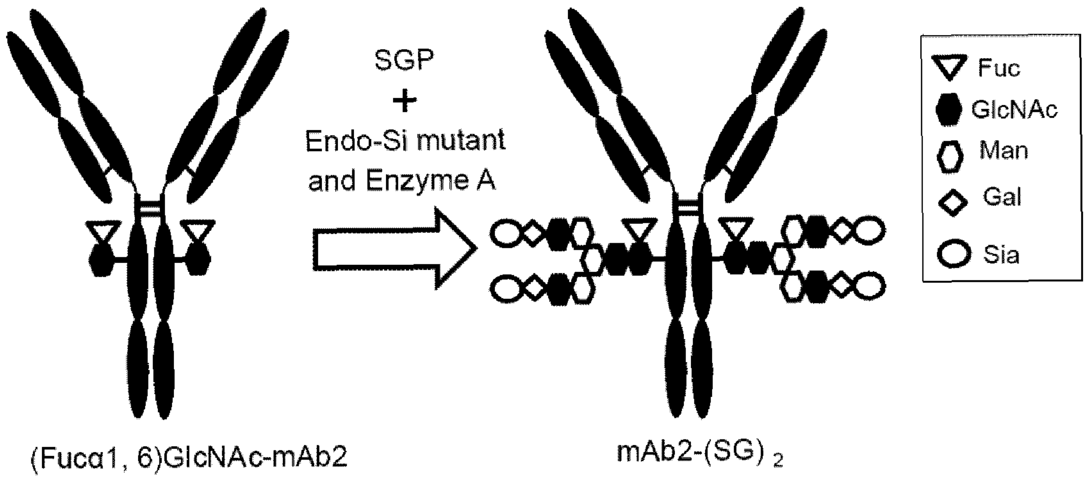
SGP
+
Endo-Si mutant
and Enzyme A
Fuc
GlcNAc
Man
Gal
Sia
(Fucα1, 6)GlcNAc-mAb2
mAb2-(SG) 2

[Fig. 18]

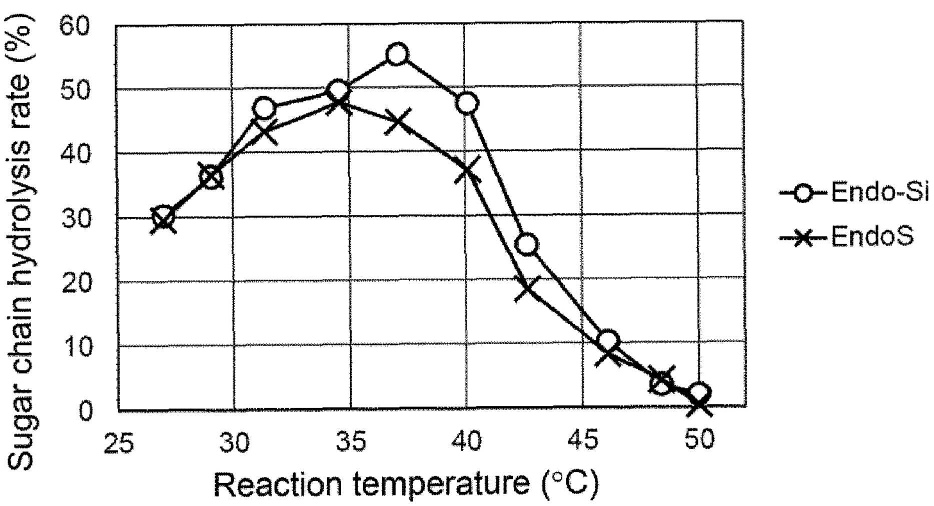
Sugar chain hydrolysis rate (%)
60
50
40
30
20
10
0
25
30
35
40
45
50
Reaction temperature (°C)
Endo-Si
EndoS

[Fig. 19]

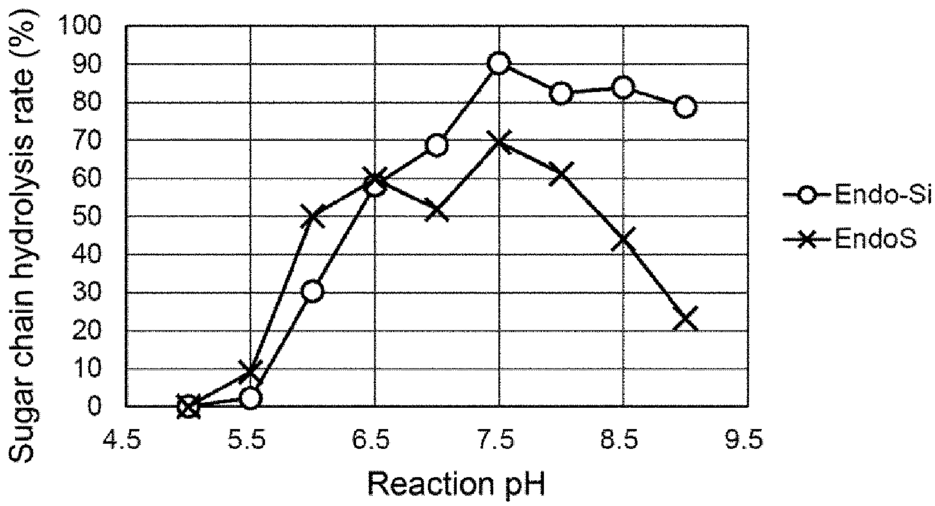
Sugar chain hydrolysis rate (%)
100
90
80
70
60
50
40
30
20
10
0
4.5
5.5
6.5
7.5
8.5
9.5
Reaction pH
Endo-Si
EndoS

[Fig. 20]

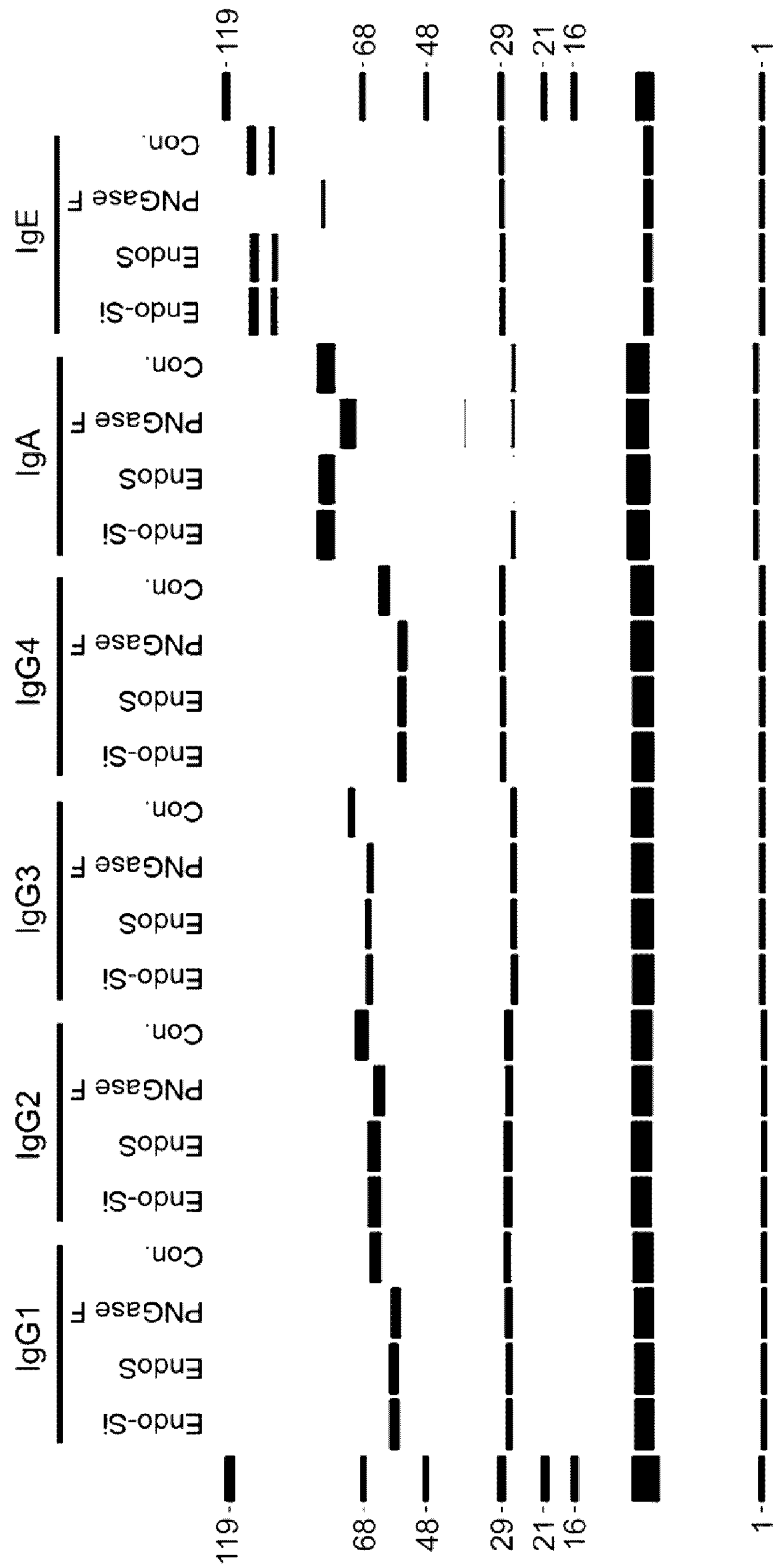

IgG1
IgG2
IgG3
IgG4
IgA
IgE
Endo-Si
EndoS
PNGase F
Con.
119
68
48
29
21
16
1

[Fig. 21]

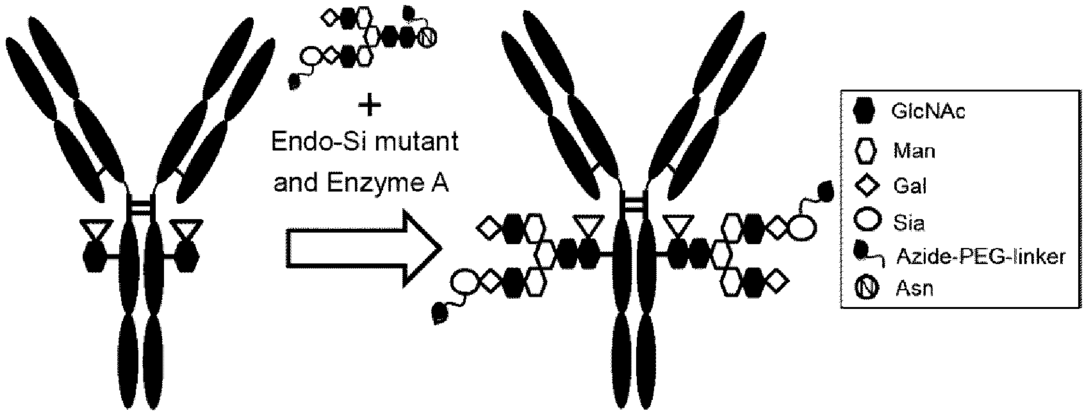


(Fucα1, 6)GlcNAc-mAb2

mAb2-(MSG1-$N_3$)$_2$

[Fig. 22]

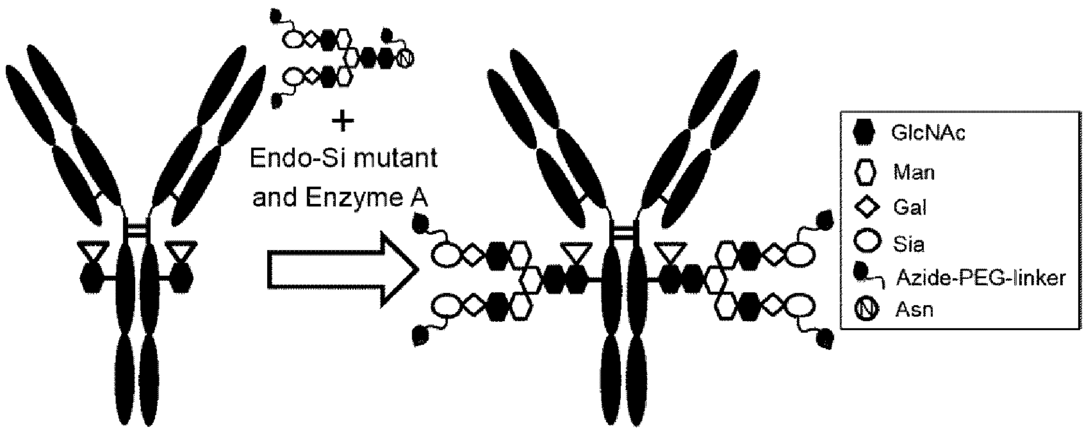


(Fucα1, 6)GlcNAc-mAb2

mAb2-[SG-($N_3$)$_2$]$_2$

**INTERNATIONAL SEARCH REPORT**

International application No.
**PCT/JP2021/032083**

**A. CLASSIFICATION OF SUBJECT MATTER**

***C07K 16/00***(2006.01)i; ***C12N 5/10***(2006.01)i; ***C12N 1/15***(2006.01)i; ***C12N 1/19***(2006.01)i; ***C12N 1/21***(2006.01)i; ***C12N 15/54***(2006.01)i; ***C12N 15/56***(2006.01)i; ***C12N 15/63***(2006.01)i; ***C12N 9/10***(2006.01)i; ***C12N 9/24***(2006.01)i; ***C12P 21/02***(2006.01)i; ***C12P 21/08***(2006.01)i

FI: C12N15/56; C12N15/63 Z; C12N1/15; C12N1/19; C12N1/21; C12N5/10; C07K16/00; C12P21/08; C12P21/02 B; C12N9/24 ZNA; C12N9/10; C12N15/54

According to International Patent Classification (IPC) or to both national classification and IPC

**B. FIELDS SEARCHED**

Minimum documentation searched (classification system followed by classification symbols)

C07K16/00; C12N5/10; C12N1/15; C12N1/19; C12N1/21; C12N15/54; C12N15/56; C12N15/63; C12N9/10; C12N9/24; C12P21/02; C12P21/08

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq; SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | SJOGREN, J. et al. EndoS2 is a unique and conserved enzyme of serotype M49 group A Streptococcus that hydrolyses N-linked glycans on IgG and α1-acid glycoprotein. Biochem. J., 2013, vol. 455, pp. 107-118<br>entire text, in particular, see page 107, abstract, page 108, right column, the last paragraph, lines 1-4, SUPPLEMENTARY ONLINE DATA, table S3 | 1, 6-16 |
| Y | | 1-44 |
| L | endo-beta-N-acetylglucosaminidase F2 [Streptococcus iniae 9117], NCBI Reference Sequence Database, Accession no. ZP_11067943, 28 November 2012, [retrieved on 28 September 2021], Retrieved from the Internet, URL, <https://www.ncbi.nlm.nih.gov/protein/ZP_11067943.1?report=girevhist> (Document cited to clarify amino acid sequence of ENDO-like protein described in the document of SJOGREN, J. et al.)<br>entire text, in particular, see columns "DEFINITION, ACCESSION, ORIGIN" | 1-44 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 October 2021** | **26 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No. PCT/JP2021/032083

C. DOCUMENTS CONSIDERED TO BE RELEVANT

| Category* | Citation of document, with indication, where appropriate, of the relevant passages | Relevant to claim No. |
|---|---|---|
| Y | TAKASHIMA, S. Analysis of EndoS2 D184M mutant for the use of glycoengineering of antibodies. 野口研究所時亮, 2017, vol. 60, pp. 25-33<br>entire text, in particular, see page 25, right column, line 11 to page 26, left column, line 4, fig. 1, page 26, right column, lines 9-12, page 27, left 3 column, paragraph [0001], page 32, left 5 column, lines 1-7, left 5 column, line 25 to right column, line 3, page 33, right column, column reference document 15, (Annual report of the Noguchi Institute) | 1-20, 27-31, 38-44 |
| Y | HUANG, W. et al. Chemoenzymatic Glycoengineering of Intact IgG Antibodies for Gain of Functions. J. Am. Chem. Soc., 2012, vol. 134, pp. 12308-12318<br>entire text, in particular, see page 12308, abstract | 1-20, 27, 42-44 |
| Y | GIDDENS, J. P. et al. Endo-F3 Glycosynthase Mutants Enable Chemoenzymatic Synthesis of Core-fucosylated Triantennary Complex Type Glycopeptides and Glycoproteins. J. Biol. Chem., 2016, vol. 291, no. 17, pp. 9356-9370<br>entire text, in particular, see page 9356, abstract, page 9361, left column, lines 23-26, fig. 2 | 1-20, 27, 42-44 |
| Y | SHIVATARE, S. S. et al. Development of glycosynthases with broad glycan specificity for the efficient glyco-remodeling of antibodies. Chem. Commun., 2018, vol. 54, pp. 6161-6164<br>entire text, in particular, see page 6162, left column, lines 42-45, the last paragraph, right column, lines 3-11, the last paragraph, lines 1-14, fig. 2a, 2b | 1-20, 27, 42-44 |
| Y | JP 2020-500549 A (CHO PHARMA INC) 16 January 2020 (2020-01-16)<br>entire text, in particular, see paragraphs [0050], [0059], [0061], fig. 3, 4 | 1-20, 27, 42-22 |
| Y | IWAMOTO, M. et al. Generation of efficient mutants of endoglycosidase from Streptococcus pyogenes and their application in a novel one-pot transglycosylation reaction for antibody modification. PLoS ONE., 2018, vol. 13, no. 2, e0193534 (pp. 1-13), Supporting information (S2 Table)<br>entire text, in particular, see, page 1, abstract, page 3, the last paragraph to page 4, line 3, fig. 3, page 4, lines 7-10, page 5, fig. 4 | 1-20, 27-31, 38-44 |
| Y | WO 2017/010559 A1 (DAIICHI SANKYO CO LTD) 19 January 2017 (2017-01-19)<br>entire text, in particular, see claims 1-3, paragraphs [0005], [0009], [0028] | 1-42 |
| Y | WO 2019/065964 A1 (DAIICHI SANKYO CO LTD) 04 April 2019 (2019-04-04)<br>entire text, in particular, see claims 30, 45, 46, 55, 57, paragraphs [0171], [0291] | 21-27, 32-42 |
| Y | WO 2020/050406 A1 (DAIICHI SANKYO CO LTD) 12 March 2020 (2020-03-12)<br>entire text, in particular, see paragraphs [0641]-[0644] | 21-27, 32-42 |
| X | PRIDGEON, J. W. et al. Complete Genome Sequence of the Attenuated Novobiocin-Resistant Streptococcus iniae Vaccine Strain ISNO. Genome Announc., 2014, vol. 2, no. 3, e00510-14 (pp. 1-2)<br>entire text, in particular, see page 1, abstract, right column, lines 10-12 | 1, 6-16 |
| X | PRIDGEON, J. W. et al. Complete Genome Sequence of a Virulent Strain, Streptococcus iniae ISET0901, solated from Diseased Tilapia. Genome Announc., 2014, vol. 2, no. 3, e00553-14 (pp. 1-2)<br>entire text, in particular, see page 1, abstract, right column, paragraph [0001] | 1, 6-16 |
| X | RAJOO, S. et al. Complete Genome Sequence of Streptococcus iniae YSFST01-82, Isolated from Olive Flounder in Jeju, South Korea. Genome Announc., 2015, vol. 3, no. 2, e00319-15 (pp. 1-2)<br>entire text, in particular, see page 1, abstract, right column, lines 8-10 | 1, 6-16 |
| X | ZHANG, B. et al. Streptococcus iniae SF1: Complete Genome Sequence, Proteomic Profile, and Immunoprotective Antigens. PLoS ONE., 2014, vol. 9, no. 3, e91324 (pp.1-14)<br>entire text, in particular, see page 1, abstract, page 2, right column, lines 10-12 | 1, 6-16 |
| X | endo-beta-N-acetylglucosaminidase [Streptococcus iniae]. GenBank., Accession no. RLU55257.1, 23 October 2018, [retrieved on 29 September 2021], Retrieved from the Internet, URL, <https://www.ncbi.nlm.nih.gov/protein/RLU55257.1?report=girevhist><br>entire text, in particular, see column "DEFINTION, ACCESSION, ORIGIN" | 1, 6-16 |

Form PCT/ISA/210 (second sheet) (January 2015)

INTERNATIONAL SEARCH REPORT

International application No.
**PCT/JP2021/032083**

**C. DOCUMENTS CONSIDERED TO BE RELEVANT**

| Category* | Citation of document, with indication, where appropriate, of the relevant passages | Relevant to claim No. |
|---|---|---|
| A | IWAMOTO, M. et al. Generation of mutants of Endo-S and their application in a novel one-pot transglycosylation reaction. 日本糖質学会年会要旨集, 2018, vol. 37th, p. 196, P-164 entire text, (The Japanese Society of Carbohydrate Research) | 1-44 |
| A | JP 2017-12152 A (INST NOGUCHI) 19 January 2017 (2017-01-19) entire text, in particular, see claim 3, paragraphs [0051], [0061] | 1-44 |
| A | LI, T. et al. Glycosynthase Mutants of Endoglycosidase S2 Show Potent Transglycosylation Activity and Remarkably Relaxed Substrate Specificity for Antibody Glycosylation Remodeling. J. Biol. Chem., 2016, vol. 291, no. 32, pp. 16508-16518 entire text, in particular, see page 16508, abstract, page 16513, left column, lines 13-17 | 1-44 |
| A | TONG, X. et al. Generation and Comparative Kinetic Analysis of New Glycosynthase Mutants from Streptococcus pyogenes Endoglycosidases for Antibody Glycoengineering. Biochemistry., 2018, vol. 57, pp. 5239-5246 entire text, in particular, see page 5239, abstract | 1-44 |
| A | JP 2019-501663 A (UNIVERSITY OF MARYLAND) 24 January 2019 (2019-01-24) entire text, in particular, see claim 1, paragraphs [0083], [0109], table 1, paragraph [0110], table 2 | 1-44 |
| A | SMITH, E. L. et al. Chemoenzymatic Fc Glycosylation via Engineered Aldehyde Tags. Bioconjugate Chem., 2014, vol. 25, pp. 788-795 entire text, in particular, see page 788, abstract | 1-44 |
| A | ZHAO, K. et al. One-step immobilization and purification of genetic engineering CBD fusion EndoS on cellulose for antibodies Fc-glycan remodeling. Bioorganic Chemistry., 2019, vol. 91, Article no. 103114 (pp. 1-8) entire text, in particular, see page 1, abstract, page 6, left column, lines 2-22, scheme 2 | 1-44 |

Form PCT/ISA/210 (second sheet) (January 2015)

**INTERNATIONAL SEARCH REPORT**

International application No.
**PCT/JP2021/032083**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2021/032083**

| Patent document cited in search report | Publication date (day/month/year) | Patent family member(s) | Publication date (day/month/year) |
|---|---|---|---|
| JP 2020-500549 A | 16 January 2020 | WO 2018/039373 A1<br>entire text, in particular, see paragraphs [0060], [0066]-[0068], fig. 3, 4<br>EP 3532090 A1<br>US 2018/0057804 A1 | |
| WO 2017/010559 A1 | 19 January 2017 | US 2018/0208915 A1<br>entire text, in particular, see claims 1-3, paragraphs [0005], [0027]-[0089]<br>EP 3323886 A1<br>JP 2017-10559 A1 | |
| WO 2019/065964 A1 | 04 April 2019 | EP 3690038 A1<br>entire text, in particular, see claims 30, 45, 46, 55, 57, paragraphs [0319], [0483]<br>US 2020/0261594 A1 | |
| WO 2020/050406 A1 | 12 March 2020 | (Family: none) | |
| JP 2017-12152 A | 19 January 2017 | (Family: none) | |
| JP 2019-501663 A | 24 January 2019 | WO 2017/124084 A1<br>entire text<br>EP 3402815 A1<br>US 2019/0194711 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018003983 A
- JP 2020022440 A
- WO 2018101451 A
- WO 2013120066 A
- WO 2017010559 A
- WO 2017124084 A
- WO 2018039373 A
- JP 2020500549 A
- JP 2020147745 A
- WO 2019065964 A
- WO 2020050406 A
- WO 2014099824 A
- WO 2014179335 A
- WO 2014189805 A
- WO 2014189806 A
- WO 2015074145 A
- WO 2015185565 A
- WO 2016096714 A
- WO 2016012305 A
- WO 2016145102 A
- WO 2017027646 A
- WO 2017027645 A
- WO 2017075477 A
- WO 2017093933 A
- WO 2017100305 A
- WO 2017123669 A
- WO 2017161349 A
- WO 2017175147 A
- WO 2017175156 A
- WO 2018009466 A
- WO 2018045204 A
- WO 2018060323 A
- WO 2018067423 A
- WO 2018065360 A
- WO 2014093936 A
- WO 2018009648 A
- WO 2018100558 A
- WO 2014057687 A
- WO 2013173496 A
- WO 2014130879 A
- WO 2017004330 A
- WO 2017004025 A
- WO 2017020972 A
- WO 2016036804 A
- WO 2015095124 A
- WO 2015052322 A
- WO 2015052534 A
- WO 2016011519 A
- WO 2015052321 A
- WO 2015031693 A
- WO 2011130613 A


### Non-patent literature cited in the description


- **ARNOLD JN et al.** *Annu Rev Immunol.,* 2007, vol. 25, 21-50
- **JEFFERIS R.** *Biotechnol Prog.,* 2005, vol. 21, 11-16
- **NIMMERJAHN F et al.** *Nat Rev Immunol.,* 2008, vol. 8, 34-47
- **JEFFERIS R.** *Nat Rev Drug Discov.,* 2009, vol. 8, 226-234
- **BUMBACA D et al.** *AAPS J.,* 2012, vol. 14, 554-558
- **ANTHONY RM et al.** *Science,* 2008, vol. 320, 373-376
- **WANG LX.** *Trends Glycosci Glycotechnol.,* 2011, vol. 23, 33-52
- **HUANG W et al.** *J Am Chem Soc.,* 2012, vol. 134, 12308-12318
- **IWAMOTO M et al.** *PLoS ONE,* 2018, vol. 13, e0193534
- **TAKEGAWA K et al.** *Biochem Int.,* 1991, vol. 24, 849-855
- **FAN SQ et al.** *J Biol Chem.,* 2012, vol. 287, 11272-11281
- **YAMAMOTO K et al.** *Biochem Biophys Res Commun.,* 1994, vol. 203, 244-252
- **ROBBINS PW et al.** *J Biol Chem.,* 1984, vol. 259, 7577-7583
- **HUANG W et al.** *Chembiochem,* 2011, vol. 12, 932-941
- **COLLIN M ; FISCHETTI VA.** *J Biol Chem.,* 2004, vol. 279, 22558-22570
- **COLLIN M ; OLSEN A.** *EMBO J.,* 2001, vol. 20, 3026-3055
- **HUANG Y et al.** *Sci Rep.,* 2018, vol. 8, 246
- **ESHIMA Y et al.** *PLoS One.,* 2015, vol. 10, e0132859
- **MURAKAMI S et al.** *Glycobiology,* 2013, vol. 23, 736-744

- **KATO T et al.** *Glycobiology,* 2002, vol. 12, 581-587
- **FUJITA K et al.** *Biosci Biotechnol Biochem.,* 2004, vol. 68, 1059-1066
- **SHADNEZHAD A et al.** *Future Microbiol.,* 2016, vol. 11, 721-736
- **FLOCK M et al.** *Infect Immun.,* 2012, vol. 80, 2914-2919
- **SJOGREN J et al.** *Biochem J.,* 2013, vol. 455, 107-118
- **GOODFELLOW JJ et al.** *J Am Chem Soc.,* 2012, vol. 134, 8030-8033
- **SHIVATARE SS et al.** *Chem Commun (Camb).,* 2018, vol. 54, 6161-6164
- **GIDDENS JP et al.** *J Biol Chem.,* 2016, vol. 291, 9356-9370
- **LI T et al.** *J Biol Chem.,* 2016, vol. 291, 16508-16518
- **PIER GB ; MADIN SH.** *Int J Syst Bacteriol.,* 1976, vol. 26, 545-553
- **YOSHIDA T.** *Fish Pathology.,* 2016, vol. 51, 44-48
- **VINCENT P et al.** *Genome Biology and Evolution,* 2014, vol. 6, 741-753
- **B. TRASTOY et al.** *PNAS,* 2014, vol. 111 (18), 6714-6719
- **ALTSCHUL, SF et al.** *Nucleic Acids Res.,* 1997, vol. 25, 3389-3402
- **UMEKAWA M. et al.** *J Biol Chem.,* 2010, vol. 285, 511-521
- **AGARD NJ et al.** *J Am Chem Soc.,* 2004, vol. 126 (46), 15046-15047